# EUROPEAN PATENT APPLICATION

(11) **EP 4 424 301 A1**
(43) Date of publication of application: **04.09.2024**
(21) Application number: 22887701.5
(22) Date of filing: 28.10.2022
(51) Int. Cl.: A61K 9/00, A61K 47/42, A61K 47/10, C12N 9/26, A61K 39/00

(54) **PHARMACEUTICAL COMPOSITION COMPRISING HUMAN HYALURONIDASE PH20 AND DRUG**

(30) Priority: 29.10.2021 KR 20210146385
(71) Applicant: Alteogen, Inc., Daejeon 34054 (KR)
(72) Inventor: PARK, Soon Jae, Daejeon 34054 (KR); KIM, Kyuwan, Daejeon 34054 (KR); NAM, Ki Seok, Daejeon 34054 (KR); SONG, Hyung-Nam, Daejeon 34054 (KR)
(74) Representative: Kilburn & Strode LLP
(86) International application number: PCT/KR2022/016709
(87) International publication number: WO 2023/075506

(57) **Abstract**

Disclosed is a pharmaceutical composition containing (a) a drug, (b) a hyaluronidase or its variant, and (c) a poloxamer-based surfactant.

The human PH20 variant contained in the pharmaceutical composition according to the present invention includes substitution of one or more amino acids at one or more regions selected from an alpha helix 8 sequence (S347 to C381) and a linker (A333 to R346) between alpha helix 7 and alpha helix 8 in a wild-type human PH20 having a sequence of SEQ ID NO: 1 and optionally includes truncation of one or more amino acids at an N-terminus and/or C-terminus in the wild-type human PH20 having a sequence of SEQ ID NO: 1. In addition, the pharmaceutical composition further contains a pharmaceutically acceptable additive, particularly a stabilizer.

Based on the effects of human PH20 or a variant thereof, the pharmaceutical composition according to the present invention can maximize the therapeutic effects of drugs used in combination therewith.

## Description

### BACKGROUND OF THE INVENTION

### Field of the Invention

The present invention relates to a pharmaceutical composition containing hyaluronidase PH20 or a variant thereof and at least one drug, and a method for treating a disease using the same, wherein the composition contains a poloxamer-based surfactant.

The pharmaceutical composition according to the present invention can be used for the treatment of various diseases, preferably for subcutaneous injection.

### Description of the Related Art

Drugs that should be administered in high doses or amounts, especially antibody drugs, are generally administered through intravenous injection, which takes about 90 minutes or more and involves additional preparation, thus disadvantageously causing inconvenience to both patients and medical staff, and entailing additional costs. On the other hand, subcutaneous injection is advantageously immediately administrated, but may cause swelling and pain at the injection site, when the amount of injection is 3~5 mL or more, due to lower absorption rate and speed compared to intravenous injection. For this reason, subcutaneous injection of protein therapeutics has been limitedly applied to injection of solutions in a small amount of 2 mL. However, when hyaluronidase is administered or injected subcutaneously in combination with therapeutic drugs, hyaluronidase hydrolyzes hyaluronic acid dispersed in the extracellular matrix, thus reducing the viscosity of the subcutaneous layer, increasing material permeability, and easily delivering high doses and large amounts of drugs into the body.

There are six types of hyaluronidase genes, namely, Hyal1, Hyal2, Hyal3, Hyal4, HyalPS1 and PH20/SPAM1, in humans. Hyal1 and Hyal2 are expressed in most tissues and PH20/SPAM1 (hereinafter referred to as "PH20") is expressed in cell membranes and acrosome membranes of sperm. HyalPS1 is not expressed as a pseudogene. PH20 is an enzyme (EC 3.2.1.35) that cleaves the β-1,4 linkage between N-acetylglucosamine, a constituent sugar of hyaluronic acid, and glucuronic acid. Human hyaluronidase PH20 has an optimum pH of 5.5, but exhibits slight activity even at a pH of 7 to 8, whereas other human hyaluronidases, including Hyal1, have an optimum pH of 3 to 4 and are less active at a pH 7 to 8. Since human subcutaneous sites have a neutral pH of about 7.4, among several types of hyaluronidases, PH20 is widely used in clinical practice. For example, PH20 is clinically applied to subcutaneous injection of antibody therapeutics, eye relaxants and anesthetic injection additives for ophthalmic surgery, hydrolysis of hyaluronic acid in the extracellular matrix of tumor cells to increase tumor cell accessibility of anticancer drugs, and to the promotion of reabsorption of excess bodily fluids and blood in tissue.

Meanwhile, currently commercially used PH20 is extracted from the testes of cattle or sheep. Examples of such PH20 include Amphadase^{®} (bovine hyaluronidase), Vitrase^{®} (sheep hyaluronidase) and the like.

It has been reported that the recombinant protein of human PH20 is expressed in yeast (P. pastoris), DS-2 insect cells, and animal cells (Chen et al., 2016, Hofinger et al., 2007). Recombinant PH20 proteins produced in insect cells and yeast differ from human PH20 in terms of the pattern of N-glycosylation during protein post-translational modification.

Although research is conducted to develop various therapeutic drugs in the form of subcutaneous injection formulations using human PH20, the problem of low stability of human PH20 itself still remains unsolved.

Under this technical background, the present inventors found that a human PH20 variant that includes at least one amino acid substitution in the alpha helix 8 region (S347 to C381), and the linker (A333 to R346) between alpha helix 7 and alpha helix 8 in the amino acid sequence of wild-type hyaluronidase PH20 and in which some of the amino acids located at the N-terminus and/or C-terminus of PH20 are truncated have excellent enzymatic activity and thermal stability and filed a patent application (PCT/KR2019/009215) based thereon.

Further, the inventors of the present application found that PH20 or a variant thereof can be applied to a drug, preferably an antibody drug, specifically a pharmaceutical composition or formulation containing a high dose of an anti-HER2 antibody or an immune checkpoint antibody, and that, in particular, when the composition or formulation contains a poloxamer-based surfactant, the activity of the drug such as the antibody drug and PH20 or a variant thereof is very stable and can be maintained for a long period of time. Based thereon, the present invention was completed.

### SUMMARY OF THE INVENTION

It is one object of the present invention to provide a novel pharmaceutical composition that contains PH20 or a variant thereof and a poloxamer-based surfactant along with a drug, wherein the thermal stability and activity of the drug and PH20 or a variant thereof can be maintained for a long period of time, especially a pharmaceutical composition applicable to subcutaneous administration.

It is another object of the present invention to provide a method of treating a disease including administering the pharmaceutical composition according to the present invention to a subject in need of treatment.

In accordance with one aspect of the present invention, the above and other objects can be accomplished by providing a pharmaceutical composition containing (a) a drug, (b) a hyaluronidase, and (c) a poloxamer-based surfactant, wherein the hyaluronidase is a wild-type PH20 having a sequence of SEQ ID NO: 1 or a variant thereof.

In the present invention, the hyaluronidase is preferably hyaluronidase PH20, particularly human PH20 or a variant thereof, but is not limited thereto, and the PH20 variant includes substitution of one or more amino acid residues selected from the group consisting of T341S, L342W, S343E, I344N, M345T, S347T, M348K, K349E, L352Q, L353A, L354I, D355K, N356E, E359D, I361T, and N363G, and further includes amino acid substitutions at one or more sites selected from the alpha helix 8 sequence (S347 to C381) and/or the linker (A333 to R346) between alpha helix 7 and alpha helix 8, wherein the PH20 or a variant thereof is characterized in that a portion of an amino acid residue located at the N-terminus and/or C-terminus is selectively truncated.

In the present invention, the hyaluronidase contained in the pharmaceutical composition maintains activity for a long period even under harsh conditions, and specifically, has a residual enzyme activity of 20% at 40 ± 2°C even after 7 days.

The pharmaceutical composition according to the present invention may further contain at least one selected from the group consisting of pharmaceutically acceptable additives, specifically buffers and stabilizers.

The pharmaceutical composition according to the present invention may be used in the form of an injectable formulation for subcutaneous administration.

### BRIEF DESCRIPTION OF THE DRAWINGS

FIG. 1 is a graph showing changes in hyaluronidase enzyme activity of Formulations 1, 2, 3, 4, and 5 using poloxamer or polysorbate as a surfactant;
FIG. 2 is a table showing the result of long-term stability test of Formulation 8;
FIG. 3 includes graphs showing changes in hyaluronidase enzyme activity of Formulations 9, 10, and 11, specifically, A of FIG. 3 is a graph showing changes in hyaluronidase enzyme activity of Formulations 9-1, 9-2, 9-3, and 9-4, B of FIG. 3 is a graph showing changes in hyaluronidase enzyme activity of Formulations 10-1, 10-2, 10-3, and 10-4, and C of FIG. 3 is a graph showing changes in hyaluronidase enzyme activity of Formulations 11-1, 11-2, 11-3, and 11-4.
FIG. 4 is a graph showing changes in hyaluronidase enzyme activity of Formulations 12-1, 12-2, 12-3, and 12-4.
FIG. 5 is a graph showing changes in hyaluronidase enzyme activity of Formulations 13-1, 13-2, 13-3, and 13-4.
FIG. 6 is a graph showing changes in hyaluronidase enzyme activity of Formulations 14-1, 14-2, 14-3, and 14-4.
FIG. 7 is a graph showing changes in hyaluronidase enzyme activity of Formulations 15-1, 15-2, 15-3, and 15-4.
FIG. 8 is a graph showing changes in hyaluronidase enzyme activity of Formulations 16-1 and 16-2.

### DETAILED DESCRIPTION OF THE INVENTION

Unless defined otherwise, all technical and scientific terms used herein have the same meanings as commonly appreciated by those skilled in the art to which the present invention pertains. In general, the nomenclature used herein is well-known and commonly used in the art.

In one aspect, the present invention is directed to a pharmaceutical composition containing (a) a drug, (b) a hyaluronidase, and (c) a poloxamer-based surfactant.

In the present invention, the hyaluronidase is derived from humans or animals, and is preferably human hyaluronidase PH20 or a variant thereof, but is not limited thereto.

The pharmaceutical composition according to the present invention can be used to prevent or treat diseases and is preferably used for subcutaneous administration.

In the present invention, the hyaluronidase contained in the pharmaceutical composition maintains activity for a long period even under harsh conditions, and specifically, has a residual enzyme activity of 20% or more at 40 ± 2°C even after 7 days.

The variant of human PH20 contained in the pharmaceutical composition according to the present invention includes substitution of some amino acid residues at a corresponding site to an alpha helix region and/or a linker region, preferably an alpha helix 8 region (S347 to C381) and/or a linker (A333 to R346) between the alpha helix 7 and alpha helix 8, more preferably, amino acid regions between T341 and N363, most preferably amino acid regions corresponding to T341 to I361, L342 to I361, S343 to I361, I344 to I361, M345 to I361, or M345 to N363, in the amino acid sequence of wild-type PH20 (having the amino acid sequence of SEQ ID NO: 1), preferably mature wild-type PH20 (having a sequence of L36 to S490 among the amino acid sequences of SEQ ID NO: 1).

As used herein, the term "mature wild-type PH20" means a protein consisting of amino acid residues L36 to S490 of SEQ ID NO: 1, which lack A491 to L509 that are not related to the substantial functions, and signal peptides M1 to T35 and PH20, in the amino acid sequence of SEQ ID NO: 1 of wild-type PH20.

**Table 1: Amino acid sequence of wild-type PH20 (SEQ. ID NO. 1)**

| |
|---|
| MGVLKFKHIFFRSFVKSSGVSQIVFTFLLIPCCLTLNFRAPPVIPNVPFLWAWNAPSE FCLGKFDEPLDMSLFSFIGSPRINATGQGVTIFYVDRLGYYPYIDSITGVTVNGGIPQ KISLQDHLDKAKKDITFYMPVDNLGMAVIDWEEWRPTWARNWKPKDVYKNRSIELVQQ QNVQLSLTEATEKAKQEFEKAGKDFLVETIKLGKLLRPNHLWGYYLFPDCYNHHYKKP GYNGSCFNVEIKRNDDLSWLWNESTALYPSIYLNTQQSPVAATLYVRNRVREAIRVSK IPDAKSPLPVFAYTRIVFTDQVLKFLSQDELVYTFGETVALGASGIVIWGTLSIMRSM KSCLLLDNYMETILNPYIINVTLAAKMCSQVLCQEQGVCIRKNWNSSDYLHLNPDNFA IQLEKGGKFTVRGKPTLEDLEQFSEKFYCSCYSTLSCKEKADVKDTDAVDVCIADGVC IDAFLKPPMETEEPQIFYNASPSTLSATMFIVSILFLIISSVASL |

As used herein, the term "PH20 variant" is intended to include a variant having not only a mutation of one or more amino acid residues, preferably substitution of one or more amino acid residues in the amino acid sequence of wild-type human PH20, but also deletion of one or more amino acid residues at the N-terminus and/or C-terminus thereof together with the substitution of the amino acid residues, and is used with substantially the same meaning as the expression "PH20 variant or fragment thereof".

In the previous research, the present inventors provide PH20 variants or fragments thereof with increased enzymatic activity and thermal stability than wild-type PH20, based on the result of test that, when some of the amino acid sequences of the alpha helix 8 region and a linker region between alpha helix 7 and alpha helix 8 of human PH20 are substituted with amino acid sequences of alpha helix 8 and the linker region between alpha helix 7 and alpha helix 8 of Hyal1, which is highly hydrophilic, the enzymatic activity and thermal stability at a neutral pH increase.

Accordingly, the PH20 variant contained in the pharmaceutical composition according to the present invention includes substitution of one or more amino acid residues of wild type PH20 (having the amino acid sequence of SEQ ID NO: 1), preferably mature wild-type PH20 (having a sequence of L36 to S490 among the amino acid sequences of SEQ ID NO: 1)selected from the group consisting of: T341S, L342W, S343E, I344N, M345T, S347T, M348K, K349E, L352Q, L353A, L354I, D355K, N356E, E359D, I361T, and N363G, more preferably, variations of one or more amino acid residues selected from the group consisting of S343E, M345T, K349E, L353A, L354I, N356E, and I361T, preferably substitution of amino acid residues, most preferably substitution of amino acid residues selected from the group consisting of L354I and N356E, and optionally includes N-terminal and/or C-terminal truncation.

In another aspect, the PH20 variant according to the present invention further includes substitution of one or more amino acid residues in the alpha helix region and/or the linker region, preferably the alpha helix 8(S347-c381) and/or the linker region between alpha helix 7 and alpha helix 8 (A333-R346) more preferably the amino acid site corresponding to T341-N363, T341-I361, L342-I361, S343-I361, I344-I361, M345-I361 or M345-N363 of the amino acid sequence of wild-type PH20 of SEQ ID NO: 1.

In particular, the PH20 variant contained in the pharmaceutical composition according to the present invention is wild type PH20, preferably the alpha helix 8 region (S347 to C381) and/or the linker region (A333 to R346) between alpha helix 7 and alpha helix 8 of the mature wild type PH20 may be substituted with some amino acid residues in the amino acid sequence of the corresponding site of Hyal1 having the sequence of SEQ ID NO: 51 (see Tables 2 and 3), but is not limited thereto.

**Table 2: Amino acid sequence of wild-type Hyal1 (SEQ. ID NO. 51)**

| |
|---|
| MAAHLLPICALFLTLLDMAQGFRGPLLPNRPFTTVWNANTQWCLERHGVDVDVSVFDV VANPGQTFRGPDMTIFYSSQLGTYPYYTPTGEPVFGGLPQNASLIAHLARTFQDILAA IPAPDFSGLAVIDWEAWRPRWAFNWDTKDIYRQRSRALVQAQHPDWPAPQVEAVAQDQ FQGAARAWMAGTLQLGRALRPRGLWGFYGFPDCYNYDFLSPNYTGQCPSGIRAQNDQL GWLWGQSRALYPSIYMPAVLEGTGKSQMYVQHRVAEAFRVAVAAGDPNLPVLPYVQIF |
| YDTTNHFLPLDELEHSLGESAAQGAAGVVLWVSWENTRTKESCQAIKEYMDTTLGPFI LNVTSGALLCSQALCSGHGRCVRRTSHPKALLLLNPASFSIQLTPGGGPLSLRGALSL EDQAQMAVEFKCRCYPGWQAPWCERKSMW |

**Table 3. Comparison in alpha helices and amino acid sequences between PH20 and Hyal1**

| Alpha helix | Amino acid sequence of PH20 | Amino acid sequence of Hyal1 |
|---|---|---|
| Alpha helix 1 | P56∼D65 | N39∼G48 |
| Alpha helix 3 | S119∼M135 | S101∼I117 |
| Alpha helix 4' | K161∼N176 | K144∼H159 |
| Alpha helix 4 | S180-R211 | P163∼R194 |
| Alpha helix 5 | F239∼S256 | P222∼S239 |
| Alpha helix 6 | A274∼D293 | K257∼G277 |
| Alpha helix 7 | S317∼G332 | P299∼G314 |
| Alpha helix 8 | S347∼C381 | T329∼C363 |

More specifically, the PH20 variant or fragment thereof contained in the pharmaceutical composition according to the present invention includes substitution of amino acid residues of L354I and/or N356E in the amino acid sequence of wild-type PH20, preferably of mature wild-type PH20, and further includes substitution of amino acid residues at one or more positions between T341 and N363, in particular at one or more positions selected from the group consisting of T341, L342, S343, I344, M345, S347, M348, K349, L352, L353, D355, E359, I361, and N363, but is not limited thereto, and more preferably, further includes substitution of one or more amino acid residues selected from the group consisting of T341S, L342W, S343E, I344N, M345T, S347T, M348K, K349E, L352Q, L353A, D355K, E359D, I361T and N363G, but is not limited thereto.

Preferably, the PH20 variant or fragment thereof contained in the pharmaceutical composition according to the present invention includes substitution of amino acid residues selected from the group consisting of M345T, S347T, M348K, K349E, L352Q, L353A, L354I, D355K, N356E, E359D, and I361T, and further includes, but are not limited to, substitution of one or more amino acid residues selected from the group consisting of T341S, L342W, S343E, I344N, and N363G.

More preferably, the PH20 variant or fragment thereof contained in the pharmaceutical composition according to the present invention may include substitution groups of any one selected from the group consisting of the following amino acid residues, but not limited thereto:
(a) T341S, L342W, S343E, I344N, M345T, S347T, M348K, K349E, L352Q, L353A, L354I, D355K, N356E, E359D and I361T;
(b) L342W, S343E, I344N, M345T, S347T, M348K, K349E, L352Q, L353A, L354I, D355K, N356E, E359D and I361T;
(c) M345T, S347T, M348K, K349E, L352Q, L353A, L354I, D355K, N356E, E359D and I361T;
(d) M345T, S347T, M348K, K349E, L352Q, L353A, L354I, D355K, N356E, E359D, I361T, and N363G;
(e) I344N, M345T, S347T, M348K, K349E, L352Q, L353A, L354I, D355K, N356E, E359D and I361T; and
(f) S343E, I344N, M345T, S347T, M348K, K349E, L352Q, L353A, L354I, D355K, N356E, E359D and I361T.

In the present invention, an expression described by a one-letter amino acid residue code together with numbers, such as "S347", means the amino acid residue at each position in the amino acid sequence of SEQ ID NO: 1.

For example, "S347" means that the amino acid residue at position 347 in the amino acid sequence of SEQ ID NO: 1 is serine and "S347T" means that serine at position 347 of SEQ ID NO: 1 is substituted with threonine.

The PH20 variant contained in the pharmaceutical composition according to the present invention is interpreted as including variants in which an amino acid residue at a specific amino acid residue position is conservatively substituted.

As used herein, the term "conservative substitution" refers to a modification of a PH20 variant that includes the substitution of one or more amino acids with other amino acids having similar biochemical properties that do not result in loss of the biological or biochemical function of the PH20 variant.

The term "conservative amino acid substitution" refers to substitution of an amino acid residue with an amino acid residue having a similar side chain. Series of amino acid residues having similar side chains have been defined and are well known in the art to which the present invention pertains. These series include amino acids with basic side chains (e.g., lysine, arginine and histidine), amino acids with acidic side chains (e.g., aspartic acid and glutamic acid), amino acids with uncharged polar side chains (e.g., glycine, asparagine, glutamine, serine, threonine, tyrosine, and cysteine), amino acids with nonpolar side chains (e.g., alanine, valine, leucine, isoleucine, proline, phenylalanine, methionine, and tryptophan), amino acids with beta-branched side chains (e.g., threonine, valine, and isoleucine), and amino acids with aromatic side chains (e.g., tyrosine, phenylalanine, tryptophan, and histidine).

It is found that the PH20 variant contained in the pharmaceutical composition according to the present invention is predicted to retain the activity thereof despite having conservative amino acid substitutions.

In addition, the PH20 variant or fragment thereof contained in the pharmaceutical composition according to the present invention is interpreted to include PH20 variants or fragments thereof having substantially the same function and/or effect as those/that of the PH20 variant or fragment thereof according to the present invention, and having amino acid sequence homology of at least 80% or 85%, preferably at least 90%, more preferably at least 95%, and most preferably at least 99% to the PH20 variant or fragment thereof according to the present invention.

The PH20 variants according to the present invention have increased expression levels and protein refolding rate, and thereby have higher thermal stability than mature wild-type PH20. Furthermore, the enzymatic activity of the PH20 variants was greater than or similar to that of mature wild-type PH20 despite the increase in thermal stability.

Meanwhile, although the mature wild-type PH20 variant having truncation of some amino acid residues such as S490 at the C-terminus is known to have decreased enzymatic activity, the PH20 variants according to the present invention exhibit increased thermostability and increased or maintained enzymatic activity compared to the mature wild-type PH20 even though they have a sequence with further truncation at the C-terminus of the mature wild-type PH20, and additionally, even though they have a sequence with truncation of five amino acids at the N-terminus of the mature wild-type PH20, the enzymatic activity was maintained, which means that the residues from P41 at the N-terminus play a key role in protein expression and enzymatic activity.

Accordingly, the PH20 variant contained in the pharmaceutical composition according to the present invention is characterized in that it includes substitutions of amino acid residues at the alpha helix 8 (S347 to C381) and/or the linker (A333 to R346) between alpha helix 7 and alpha helix 8, and further truncation of some amino acid residues at the C- and/or N-terminus, but is not limited thereto.

In one embodiment, the PH20 or PH20 variant contained in the pharmaceutical composition according to the present invention may be characterized in which truncation of the amino acid sequence of SEQ ID NO: 1 occurs before an amino acid residue selected from the group consisting of M1 to P42 at the N-terminus, preferably before an amino acid residue L36, N37, F38, R39, A40, P41, or P42 at the N-terminus, so that one or more amino acid residues at the N-terminus are deleted, and/or truncation occurs after an amino acid residue selected from the group consisting of V455 to W509, preferably after an amino acid residue selected from the group consisting of V455 to S490, most preferably after an amino acid residue V455, C458, D461, C464, 1465, D466, A467, F468, K470, P471, P472, M473, E474, T475, E476, P478, I480, Y482, A484, P486, T488, or S490 at the C-terminus, so that one or more amino acid residues at the C-terminus are deleted.

The expression "truncation occurs before an amino acid residue L36, N37, F38, R39, A40, P41, or P42" means that all amino acid residues from M1 to T35 immediately before L36, all amino acid residues from M1 to L36 immediately before N37, all amino acid residues from M1 to N37 immediately before F38, all amino acid residues from M1 to F38 immediately before R39, all amino acid residues from M1 to R39 immediately before A40, all amino acid residues from M1 to A40 immediately before P41, or all amino acid residues from M1 to P41 immediately before P42 in the amino acid sequence of SEQ ID NO: 1 are truncated and removed. The expression "truncation occurs before M1 at the N-terminus of SEQ ID NO: 1" means that no truncation occurs at the N-terminus.

Additionally, the expression "truncation occurs after V455, C458, D461, C464, 1465, D466, A467, F468, K470, P471, P472, M473, E474, T475, E476, P478, I480, Y482, A484, P486, T488 or S490 at the C-terminus" means that truncation and deletion occur from an amino acid residue after the amino acid residue V455, D456, C458, D461, C464, 1465, D466, A467, F468, K470, P471, P472, M473, E474, T475, E476, P478, I480, Y482, A484, P486, T488 or S490 in the amino acid sequence of SEQ ID NO: 1. For example, the expression "truncation occurs after S490" means that truncation occurs between S490 and A491.

Preferably, the human PH20 variant contained in the pharmaceutical composition according to the present invention may include an amino acid sequence selected from the group consisting of the amino acid sequences of SEQ ID NO: 5 to SEQ ID NO: 50, more preferably an amino acid sequence of SEQ ID NO: 44, but is not limited thereto. The amino acid sequences substituted or truncated in PH20 variants prepared in specific examples according to the present invention are shown in Table 4.

**Table 4. Amino acid sequences of PH20 variants according to the present invention and substitution/truncation characteristics thereof**

| Name | SEQ ID NO. | Substitution | Sequence |
|---|---|---|---|
| HM1 | 5 | Substitution of 12 amino acids with M345T, S347T, M348K, K349E, L352Q, L353A, L354I, D355K, N356E, E359D, I361T, and N363G. | LNFRAPPVIPNVPFLWAWNAPSEFCLGKFDEP LDMSLFSFIGSPRINATGQGVTIFYVDRLGYYP YIDSITGVTVNGGIPQKISLQDHLDKAKKDITF YMPVDNLGMAVIDWEEWRPTWARNWKPKD VYKNRSIELVQQQNVQLSLTEATEKAKQEFEK AGKDFLVETIKLGKLLRPNHLWGYYLFPDCYN HHYKKPGYNGSCFNVEIKRNDDLSWLWNEST ALYPSIYLNTQQSPVAATLYVRNRVREAIRVSK IPDAKSPLPVFAYTRIVFTDQVLKFLSQDELVY TFGETVALGASGIVIWGTLSITRTKESCQAIKE YMDTTLGPYIINVTLAAKMCSQVLCQEQGVCI RKNWNSSDYLHLNPDNFAIQLEKGGKFTVRG KPTLEDLEQFSEKFYCSCYSTLSCKEKADVKD TDAVDVCIADGVCIDAFLKPPMETEEPQIFYNA |
| | | | SPSTLS |
| HM2 | 6 | Substitution of 7 amino acids with Y365F, I367L, L371S, A372G, K374L, M375L, and V379A. | LNFRAPPVIPNVPFLWAWNAPSEFCLGKFDEP LDMSLFSFIGSPRINATGQGVTIFYVDRLGYYP YIDSITGVTVNGGIPQKISLQDHLDKAKKDITF YMPVDNLGMAVIDWEEWRPTWARNWKPKD VYKNRSIELVQQQNVQLSLTEATEKAKQEFEK AGKDFLVETIKLGKLLRPNHLWGYYLFPDCYN HHYKKPGYNGSCFNVEIKRNDDLSWLWNEST ALYPSIYLNTQQSPVAATLYVRNRVREAIRVSK IPDAKSPLPVFAYTRIVFTDQVLKFLSQDELVY TFGETVALGASGIVIWGTLSITRTKESCQAIKEY MDTTLNPFILNVTSGALLCSQALCQEQGVCIR KNWNSSDYLHLNPDNFAIQLEKGGKFTVRGKP TLEDLEQFSEKFYCSCYSTLSCKEKADVKDTD AVDVCIADGVCIDAFLKPPMETEEPQIFYNASP STLS |
| HM3 | 7 | Substitution of 19 amino acids with M345T, S347T, M348K, K349E, L352Q, L353A, L354I, D355K, N356E, E359D, I361T, N363G, Y365F, I367L, L371S, A372G, K374L, M375L, and V379A. | LNFRAPPVIPNVPFLWAWNAPSEFCLGKFDEP LDMSLFSFIGSPRINATGQGVTIFYVDRLGYYP YIDSITGVTVNGGIPQKISLQDHLDKAKKDITF YMPVDNLGMAVIDWEEWRPTWARNWKPKD VYKNRSIELVQQQNVQLSLTEATEKAKQEFEK AGKDFLVETIKLGKLLRPNHLWGYYLFPDCYN HHYKKPGYNGSCFNVEIKRNDDLSWLWNEST ALYPSIYLNTQQSPVAATLYVRNRVREAIRVSK IPDAKSPLPVFAYTRIVFTDQVLKFLSQDELVY TFGETVALGASGIVIWGTLSITRTKESCQAIKE YMDTTLGPFILNVTSGALLCSQALCQEQGVCI RKNWNSSDYLHLNPDNFAIQLEKGGKFTVRG KPTLEDLEQFSEKFYCSCYSTLSCKEKADVKD TDAVDVCIADGVCIDAFLKPPMETEEPQIFYNA SPSTLS |
| HM4 | 8 | Substitution of 17 amino acids with G340V, T341S, L342W, S343E, I344N, M345T, S347T, M348K, K349E, L352Q, L353A, L354I, D355K, N356E, E359D, I361T, and N363G. | LNFRAPPVIPNVPFLWAWNAPSEFCLGKFDEP LDMSLFSFIGSPRINATGQGVTIFYVDRLGYYP YIDSITGVTVNGGIPQKISLQDHLDKAKKDITF |
| | | | YMPVDNLGMAVIDWEEWRPTWARNWKPKD VYKNRSIELVQQQNVQLSLTEATEKAKQEFEK AGKDFLVETIKLGKLLRPNHLWGYYLFPDCYN HHYKKPGYNGSCFNVEIKRNDDLSWLWNEST ALYPSIYLNTQQSPVAATLYVRNRVREAIRVSK IPDAKSPLPVFAYTRIVFTDQVLKFLSQDELVY TFGETVALGASGIVIWVSWENTRTKESCQAIK EYMDTTLGPYIINVTLAAKMCSQVLCQEQGV CIRKNWNSSDYLHLNPDNFAIQLEKGGKFTVR GKPTLEDLEQFSEKFYCSCYSTLSCKEKADVK DTDAVDVCIADGVCIDAFLKPPMETEEPQIFYN ASPSTLS |
| HM6 | 9 | Substitution of 11 amino acids with M345T, S347T, M348K, K349E, L352Q, L353A, L354I, D355K, N356E, E359D, and I361T. | LNFRAPPVIPNVPFLWAWNAPSEFCLGKFDEP LDMSLFSFIGSPRINATGQGVTIFYVDRLGYYP YIDSITGVTVNGGIPQKISLQDHLDKAKKDITF YMPVDNLGMAVIDWEEWRPTWARNWKPKD VYKNRSIELVQQQNVQLSLTEATEKAKQEFEK AGKDFLVETIKLGKLLRPNHLWGYYLFPDCYN HHYKKPGYNGSCFNVEIKRNDDLSWLWNEST ALYPSIYLNTQQSPVAATLYVRNRVREAIRVSK IPDAKSPLPVFAYTRIVFTDQVLKFLSQDELVY TFGETVALGASGIVIWGTLSITRTKESCQAIKE YMDTTLNPYIINVTLAAKMCSQVLCQEQGVCI RKNWNSSDYLHLNPDNFAIQLEKGGKFTVRG KPTLEDLEQFSEKFYCSCYSTLSCKEKADVKD TDAVDVCIADGVCIDAFLKPPMETEEPQIFYNA SPSTLS |
| HM7 | 10 | Substitution of 16 amino acids with G340V, T341S L342W, S343E, I344N, M345T, S347T, M348K, K349E, L352Q, L353A, L354I, D355K, N356E, E359D, and I361T. | LNFRAPPVIPNVPFLWAWNAPSEFCLGKFDEP LDMSLFSFIGSPRINATGQGVTIFYVDRLGYYP YIDSITGVTVNGGIPQKISLQDHLDKAKKDITF YMPVDNLGMAVIDWEEWRPTWARNWKPKD VYKNRSIELVQQQNVQLSLTEATEKAKQEFEK AGKDFLVETIKLGKLLRPNHLWGYYLFPDCYN HHYKKPGYNGSCFNVEIKRNDDLSWLWNEST |
| | | | ALYPSIYLNTQQSPVAATLYVRNRVREAIRVSK IPDAKSPLPVFAYTRIVFTDQVLKFLSQDELVY TFGETVALGASGIVIWVSWENTRTKESCQAIK EYMDTTLNPYIINVTLAAKMCSQVLCQEQGV CIRKNWNSSDYLHLNPDNFAIQLEKGGKFTVR GKPTLEDLEQFSEKFYCSCYSTLSCKEKADVK DTDAVDVCIADGVCIDAFLKPPMETEEPQIFYN ASPSTLS |
| HM8 | 11 | Substitution of 12 amino acids with I344N, M345T, S347T, M348K, K349E, L352Q, L353A, L354I, D355K, N356E, E359D, and I361T. | LNFRAPPVIPNVPFLWAWNAPSEFCLGKFDEP LDMSLFSFIGSPRINATGQGVTIFYVDRLGYYP YIDSITGVTVNGGIPQKISLQDHLDKAKKDITF YMPVDNLGMAVIDWEEWRPTWARNWKPKD VYKNRSIELVQQQNVQLSLTEATEKAKQEFEK AGKDFLVETIKLGKLLRPNHLWGYYLFPDCYN HHYKKPGYNGSCFNVEIKRNDDLSWLWNEST ALYPSIYLNTQQSPVAATLYVRNRVREAIRVSK IPDAKSPLPVFAYTRIVFTDQVLKFLSQDELVY TFGETVALGASGIVIWGTLSNTRTKESCQAIK EYMDTTLNPYIINVTLAAKMCSQVLCQEQGV CIRKNWNSSDYLHLNPDNFAIQLEKGGKFTVR GKPTLEDLEQFSEKFYCSCYSTLSCKEKADVK DTDAVDVCIADGVCIDAFLKPPMETEEPQIFYN ASPSTLS |
| HM9 | 12 | Substitution of 13 amino acids with S343E, I344N, M345T, S347T, M348K, K349E, L352Q, L353A, L354I, D355K, N356E, E359D, and I361T. | LNFRAPPVIPNVPFLWAWNAPSEFCLGKFDEP LDMSLFSFIGSPRINATGQGVTIFYVDRLGYYP YIDSITGVTVNGGIPQKISLQDHLDKAKKDITF YMPVDNLGMAVIDWEEWRPTWARNWKPKD VYKNRSIELVQQQNVQLSLTEATEKAKQEFEK AGKDFLVETIKLGKLLRPNHLWGYYLFPDCYN HHYKKPGYNGSCFNVEIKRNDDLSWLWNEST ALYPSIYLNTQQSPVAATLYVRNRVREAIRVSK IPDAKSPLPVFAYTRIVFTDQVLKFLSQDELVY TFGETVALGASGIVIWGTLENTRTKESCQAIK EYMDTTLNPYIINVTLAAKMCSQVLCQEQGV |
| | | | CIRKNWNSSDYLHLNPDNFAIQLEKGGKFTVR GKPTLEDLEQFSEKFYCSCYSTLSCKEKADVK DTDAVDVCIADGVCIDAFLKPPMETEEPQIFYN ASPSTLS |
| HM10 | 13 | Substitution of 14 amino acids with L342W, S343E, I344N, M345T, S347T, M348K, K349E, L352Q, L353A, L354I, D355K, N356E, E359D, and I361T. | LNFRAPPVIPNVPFLWAWNAPSEFCLGKFDEP LDMSLFSFIGSPRINATGQGVTIFYVDRLGYYP YIDSITGVTVNGGIPQKISLQDHLDKAKKDITF YMPVDNLGMAVIDWEEWRPTWARNWKPKD VYKNRSIELVQQQNVQLSLTEATEKAKQEFEK AGKDFLVETIKLGKLLRPNHLWGYYLFPDCYN HHYKKPGYNGSCFNVEIKRNDDLSWLWNEST ALYPSIYLNTQQSPVAATLYVRNRVREAIRVSK IPDAKSPLPVFAYTRIVFTDQVLKFLSQDELVY TFGETVALGASGIVIWGTWENTRTKESCQAIK EYMDTTLNPYIINVTLAAKMCSQVLCQEQGV CIRKNWNSSDYLHLNPDNFAIQLEKGGKFTVR GKPTLEDLEQFSEKFYCSCYSTLSCKEKADVK DTDAVDVCIADGVCIDAFLKPPMETEEPQIFYN ASPSTLS |
| HM11 | 14 | Substitution of 13 amino acids with M345T, S347T, M348K, K349E, L352Q, L353A, L354I, D355K, N356E, E359D, I361T, Y365F, and I367L | LNFRAPPVIPNVPFLWAWNAPSEFCLGKFDEP LDMSLFSFIGSPRINATGQGVTIFYVDRLGYYP YIDSITGVTVNGGIPQKISLQDHLDKAKKDITF YMPVDNLGMAVIDWEEWRPTWARNWKPKD VYKNRSIELVQQQNVQLSLTEATEKAKQEFEK AGKDFLVETIKLGKLLRPNHLWGYYLFPDCYN HHYKKPGYNGSCFNVEIKRNDDLSWLWNEST ALYPSIYLNTQQSPVAATLYVRNRVREAIRVSK IPDAKSPLPVFAYTRIVFTDQVLKFLSQDELVY TFGETVALGASGIVIWGTLSITRTKESCQAIKE YMDTTLNPFILNVTLAAKMCSQVLCQEQGVCI RKNWNSSDYLHLNPDNFAIQLEKGGKFTVRG KPTLEDLEQFSEKFYCSCYSTLSCKEKADVKD TDAVDVCIADGVCIDAFLKPPMETEEPQIFYNA SPSTLS |
| HM12 | 15 | Substitution of 15 amino acids with M345T, S347T, M348K, K349E, L352Q, L353A, L3541, D355K, N356E, E359D, I361T, Y365F, I367L, L371S, and A372G. | LNFRAPPVIPNVPFLWAWNAPSEFCLGKFDEP LDMSLFSFIGSPRINATGQGVTIFYVDRLGYYP YIDSITGVTVNGGIPQKISLQDHLDKAKKDITF YMPVDNLGMAVIDWEEWRPTWARNWKPKD VYKNRSIELVQQQNVQLSLTEATEKAKQEFEK AGKDFLVETIKLGKLLRPNHLWGYYLFPDCYN HHYKKPGYNGSCFNVEIKRNDDLSWLWNEST ALYPSIYLNTQQSPVAATLYVRNRVREAIRVSK IPDAKSPLPVFAYTRIVFTDQVLKFLSQDELVY TFGETVALGASGIVIWGTLSITRTKESCQAIKE YMDTTLNPFILNVTSGAKMCSQVLCQEQGVCI RKNWNSSDYLHLNPDNFAIQLEKGGKFTVRG KPTLEDLEQFSEKFYCSCYSTLSCKEKADVKD TDAVDVCIADGVCIDAFLKPPMETEEPQIFYNA SPSTLS |
| HM13 | 16 | Substitution of 11 amino acids with M345T, S347T, M348K, K349E, L352Q, L353A, L3541, D355K, N356E, E359D, and I361T, and truncation before residue F38 at the N-terminus. | FRAPPVIPNVPFLWAWNAPSEFCLGKFDEPLD MSLFSFIGSPRINATGQGVTIFYVDRLGYYPYID SITGVTVNGGIPQKISLQDHLDKAKKDITFYMP VDNLGMAVIDWEEWRPTWARNWKPKDVYK NRSIELVQQQNVQLSLTEATEKAKQEFEKAGK DFLVETIKLGKLLRPNHLWGYYLFPDCYNHHY KKPGYNGSCFNVEIKRNDDLSWLWNESTALY PSIYLNTQQSPVAATLYVRNRVREAIRVSKIPD AKSPLPVFAYTRIVFTDQVLKFLSQDELVYTFG ETVALGASGIVIWGTLSITRTKESCQAIKEYM DTTLNPYIINVTLAAKMCSQVLCQEQGVCIRK NWNSSDYLHLNPDNFAIQLEKGGKFTVRGKPT LEDLEQFSEKFYCSCYSTLSCKEKADVKDTDA VDVCIADGVCIDAFLKPPMETEEPQIFYNASPS TLS |
| HM14 | 17 | Substitution of 11 amino acids with M345T, S347T, M348K, K349E, L352Q, L353A, L3541, D355K, N356E, E359D, and I361T, and truncation after carboxy of 1465. | LNFRAPPVIPNVPFLWAWNAPSEFCLGKFDEP LDMSLFSFIGSPRINATGQGVTIFYVDRLGYYP YIDSITGVTVNGGIPQKISLQDHLDKAKKDITF YMPVDNLGMAVIDWEEWRPTWARNWKPKD |
| | | | VYKNRSIELVQQQNVQLSLTEATEKAKQEFEK AGKDFLVETIKLGKLLRPNHLWGYYLFPDCYN HHYKKPGYNGSCFNVEIKRNDDLSWLWNEST ALYPSIYLNTQQSPVAATLYVRNRVREAIRVSK IPDAKSPLPVFAYTRIVFTDQVLKFLSQDELVY TFGETVALGASGIVIWGTLSITRTKESCQAIKE YMDTTLNPYIINVTLAAKMCSQVLCQEQGVCI RKNWNSSDYLHLNPDNFAIQLEKGGKFTVRG KPTLEDLEQFSEKFYCSCYSTLSCKEKADVKD TDAVDVCIADGVCI |
| HM15 | 18 | Substitution of 11 amino acids with M345T, S347T, M348K, K349E, L352Q, L353A, L3541, D355K, N356E, E359D, and I361T, and truncation after carboxy of F468. | LNFRAPPVIPNVPFLWAWNAPSEFCLGKFDEP LDMSLFSFIGSPRINATGQGVTIFYVDRLGYYP YIDSITGVTVNGGIPQKISLQDHLDKAKKDITF YMPVDNLGMAVIDWEEWRPTWARNWKPKD VYKNRSIELVQQQNVQLSLTEATEKAKQEFEK AGKDFLVETIKLGKLLRPNHLWGYYLFPDCYN HHYKKPGYNGSCFNVEIKRNDDLSWLWNEST ALYPSIYLNTQQSPVAATLYVRNRVREAIRVSK IPDAKSPLPVFAYTRIVFTDQVLKFLSQDELVY TFGETVALGASGIVIWGTLSITRTKESCQAIKE YMDTTLNPYIINVTLAAKMCSQVLCQEQGVCI RKNWNSSDYLHLNPDNFAIQLEKGGKFTVRG KPTLEDLEQFSEKFYCSCYSTLSCKEKADVKD TDAVDVCIADGVCIDAF |
| HM16 | 19 | Substitution of 11 amino acids with M345T, S347T, M348K, K349E, L352Q, L353A, L354I, D355K, N356E, E359D, and I361T, and truncation after carboxy of P471. | LNFRAPPVIPNVPFLWAWNAPSEFCLGKFDEP LDMSLFSFIGSPRINATGQGVTIFYVDRLGYYP YIDSITGVTVNGGIPQKISLQDHLDKAKKDITF YMPVDNLGMAVIDWEEWRPTWARNWKPKD VYKNRSIELVQQQNVQLSLTEATEKAKQEFEK AGKDFLVETIKLGKLLRPNHLWGYYLFPDCYN HHYKKPGYNGSCFNVEIKRNDDLSWLWNEST ALYPSIYLNTQQSPVAATLYVRNRVREAIRVSK IPDAKSPLPVFAYTRIVFTDQVLKFLSQDELVY TFGETVALGASGIVIWGTLSITRTKESCQAIKE |
| | | | YMDTTLNPYIINVTLAAKMCSQVLCQEQGVCI RKNWNSSDYLHLNPDNFAIQLEKGGKFTVRG KPTLEDLEQFSEKFYCSCYSTLSCKEKADVKD TDAVDVCIADGVCIDAFLKP |
| HM17 | 20 | Substitution of amino acids L36 to V47 with FRGPLLPNR, and substitution of 11 amino acids with M345T, S347T, M348K, K349E, L352Q, L353A, L354I, D355K, N356E, E359D, and I361T. | FRGPLLPNRPFLWAWNAPSEFCLGKFDEPLD MSLFSFIGSPRINATGQGVTIFYVDRLGYYPYID SITGVTVNGGIPQKISLQDHLDKAKKDITFYMP VDNLGMAVIDWEEWRPTWARNWKPKDVYK NRSIELVQQQNVQLSLTEATEKAKQEFEKAGK DFLVETIKLGKLLRPNHLWGYYLFPDCYNHHY KKPGYNGSCFNVEIKRNDDLSWLWNESTALY PSIYLNTQQSPVAATLYVRNRVREAIRVSKIPD AKSPLPVFAYTRIVFTDQVLKFLSQDELVYTFG ETVALGASGIVIWGTLSITRTKESCQAIKEYM DTTLNPYIINVTLAAKMCSQVLCQEQGVCIRK NWNSSDYLHLNPDNFAIQLEKGGKFTVRGKPT LEDLEQFSEKFYCSCYSTLSCKEKADVKDTDA VDVCIADGVCIDAFLKPPMETEEPQIFYNASPS TLS |
| HM18 | 21 | Substitution of amino acids L36 to A52 with FRGPLLPNRPFTTV, and substitution of 11 amino acids with M345T, S347T, M348K, K349E, L352Q, L353A, L354I, D355K, N356E, E359D, and I361T. | FRGPLLPNRPFTTVWNAPSEFCLGKFDEPLD MSLFSFIGSPRINATGQGVTIFYVDRLGYYPYID SITGVTVNGGIPQKISLQDHLDKAKKDITFYMP VDNLGMAVIDWEEWRPTWARNWKPKDVYK NRSIELVQQQNVQLSLTEATEKAKQEFEKAGK DFLVETIKLGKLLRPNHLWGYYLFPDCYNHHY KKPGYNGSCFNVEIKRNDDLSWLWNESTALY PSIYLNTQQSPVAATLYVRNRVREAIRVSKIPD AKSPLPVFAYTRIVFTDQVLKFLSQDELVYTFG ETVALGASGIVIWGTLSITRTKESCQAIKEYM DTTLNPYIINVTLAAKMCSQVLCQEQGVCIRK NWNSSDYLHLNPDNFAIQLEKGGKFTVRGKPT LEDLEQFSEKFYCSCYSTLSCKEKADVKDTDA VDVCIADGVCIDAFLKPPMETEEPQIFYNASPS TLS |
| HM19 | 22 | Substitution of 14 amino acids with L342W, S343E, I344N, M345T, S347T, M348K, K349E, L352Q, L353A, L354I, D355K, N356E, E359D, and I361T, truncation before residue F38 at the N-terminus, and truncation after residue K470 at the C-terminus | FRAPPVIPNVPFLWAWNAPSEFCLGKFDEPLD MSLFSFIGSPRINATGQGVTIFYVDRLGYYPYID SITGVTVNGGIPQKISLQDHLDKAKKDITFYMP VDNLGMAVIDWEEWRPTWARNWKPKDVYK NRSIELVQQQNVQLSLTEATEKAKQEFEKAGK DFLVETIKLGKLLRPNHLWGYYLFPDCYNHHY KKPGYNGSCFNVEIKRNDDLSWLWNESTALY PSIYLNTQQSPVAATLYVRNRVREAIRVSKIPD AKSPLPVFAYTRIVFTDQVLKFLSQDELVYTFG ETVALGASGIVIWGTWENTRTKESCQAIKEY MDTTLNPYIINVTLAAKMCSQVLCQEQGVCIR KNWNSSDYLHLNPDNFAIQLEKGGKFTVRGKP TLEDLEQFSEKFYCSCYSTLSCKEKADVKDTD AVDVCIADGVCIDAFLK |
| HM20 | 23 | Substitution of 14 amino acids with L342W, S343E, I344N, M345T, S347T, M348K, K349E, L352Q, L353A, L354I, D355K, N356E, E359D, and I361T, truncation before residue F38 at the N-terminus, and truncation after residue F468 at the C-terminus | FRAPPVIPNVPFLWAWNAPSEFCLGKFDEPLD MSLFSFIGSPRINATGQGVTIFYVDRLGYYPYID SITGVTVNGGIPQKISLQDHLDKAKKDITFYMP VDNLGMAVIDWEEWRPTWARNWKPKDVYK NRSIELVQQQNVQLSLTEATEKAKQEFEKAGK DFLVETIKLGKLLRPNHLWGYYLFPDCYNHHY KKPGYNGSCFNVEIKRNDDLSWLWNESTALY PSIYLNTQQSPVAATLYVRNRVREAIRVSKIPD AKSPLPVFAYTRIVFTDQVLKFLSQDELVYTFG ETVALGASGIVIWGTWENTRTKESCQAIKEY MDTTLNPYIINVTLAAKMCSQVLCQEQGVCIR KNWNSSDYLHLNPDNFAIQLEKGGKFTVRGKP TLEDLEQFSEKFYCSCYSTLSCKEKADVKDTD AVDVCIADGVCIDAF |
| HM21 | 24 | Substitution of 15 amino acids with T341S, L342W, S343E, I344N, M345T, S347T, M348K, K349E, L352Q, L353A, L354I, D355K, N356E, E359D, and I361T. | LNFRAPPVIPNVPFLWAWNAPSEFCLGKFDEP LDMSLFSFIGSPRINATGQGVTIFYVDRLGYYP YIDSITGVTVNGGIPQKISLQDHLDKAKKDITF YMPVDNLGMAVIDWEEWRPTWARNWKPKD VYKNRSIELVQQQNVQLSLTEATEKAKQEFEK AGKDFLVETIKLGKLLRPNHLWGYYLFPDCYN |
| | | | HHYKKPGYNGSCFNVEIKRNDDLSWLWNEST ALYPSIYLNTQQSPVAATLYVRNRVREAIRVSK IPDAKSPLPVFAYTRIVFTDQVLKFLSQDELVY TFGETVALGASGIVIWGSWENTRTKESCQAIK EYMDTTLNPYIINVTLAAKMCSQVLCQEQGV CIRKNWNSSDYLHLNPDNFAIQLEKGGKFTVR GKPTLEDLEQFSEKFYCSCYSTLSCKEKADVK DTDAVDVCIADGVCIDAFLKPPMETEEPQIFYN ASPSTLS |
| HM24 | 25 | Substitution of 11 amino acids with M345T, S347T, M348K, K349E, L352Q, L353A, L3541, D355K, N356E, E359D, and I361T, and truncation before residue A40 at the N-terminus. | APPVIPNVPFLWAWNAPSEFCLGKFDEPLDMS LFSFIGSPRINATGQGVTIFYVDRLGYYPYIDSIT GVTVNGGIPQKISLQDHLDKAKKDITFYMPVD NLGMAVIDWEEWRPTWARNWKPKDVYKNRS IELVQQQNVQLSLTEATEKAKQEFEKAGKDFL VETIKLGKLLRPNHLWGYYLFPDCYNHHYKK PGYNGSCFNVEIKRNDDLSWLWNESTALYPSI YLNTQQSPVAATLYVRNRVREAIRVSKIPDAK SPLPVFAYTRIVFTDQVLKFLSQDELVYTFGET VALGASGIVIWGTLSITRTKESCQAIKEYMDT TLNPYIINVTLAAKMCSQVLCQEQGVCIRKNW NSSDYLHLNPDNFAIQLEKGGKFTVRGKPTLE DLEQFSEKFYCSCYSTLSCKEKADVKDTDAVD VCIADGVCIDAFLKPPMETEEPQIFYNASPSTLS |
| HM25 | 26 | Substitution of 11 amino acids with M345T, S347T, M348K, K349E, L352Q, L353A, L3541, D355K, N356E, E359D, and I361T, and truncation before residue P42 at the N-terminus. | PVIPNVPFLWAWNAPSEFCLGKFDEPLDMSLF SFIGSPRINATGQGVTIFYVDRLGYYPYIDSITG VTVNGGIPQKISLQDHLDKAKKDITFYMPVDN LGMAVIDWEEWRPTWARNWKPKDVYKNRSI ELVQQQNVQLSLTEATEKAKQEFEKAGKDFL VETIKLGKLLRPNHLWGYYLFPDCYNHHYKK PGYNGSCFNVEIKRNDDLSWLWNESTALYPSI YLNTQQSPVAATLYVRNRVREAIRVSKIPDAK SPLPVFAYTRIVFTDQVLKFLSQDELVYTFGET VALGASGIVIWGTLSITRTKESCQAIKEYMDT TLNPYIINVTLAAKMCSQVLCQEQGVCIRKNW |
| | | | NSSDYLHLNPDNFAIQLEKGGKFTVRGKPTLE DLEQFSEKFYCSCYSTLSCKEKADVKDTDAVD VCIADGVCIDAFLKPPMETEEPQIFYNASPSTLS |
| HM29 | 27 | Substitution of 14 amino acids with L342W, S343E, I344N, M345T, S347T, M348K, K349E, L352Q, L353A, L3541, D355K, N356E, E359D, and I361T, truncation before residue L36 at the N-terminus, and truncation after residue A467 at the C-terminus. | LNFRAPPVIPNVPFLWAWNAPSEFCLGKFDEP LDMSLFSFIGSPRINATGQGVTIFYVDRLGYYP YIDSITGVTVNGGIPQKISLQDHLDKAKKDITF YMPVDNLGMAVIDWEEWRPTWARNWKPKD VYKNRSIELVQQQNVQLSLTEATEKAKQEFEK AGKDFLVETIKLGKLLRPNHLWGYYLFPDCYN HHYKKPGYNGSCFNVEIKRNDDLSWLWNEST ALYPSIYLNTQQSPVAATLYVRNRVREAIRVSK IPDAKSPLPVFAYTRIVFTDQVLKFLSQDELVY TFGETVALGASGIVIWGTWENTRTKESCQAIK EYMDTTLNPYIINVTLAAKMCSQVLCQEQGV CIRKNWNSSDYLHLNPDNFAIQLEKGGKFTVR GKPTLEDLEQFSEKFYCSCYSTLSCKEKADVK DTDAVDVCIADGVCIDA |
| HM30 | 28 | Substitution of 14 amino acids with L342W, S343E, I344N, M345T, S347T, M348K, K349E, L352Q, L353A, L3541, D355K, N356E, E359D, and I361T, truncation before residue L36 at the N-terminus, and truncation after residue C464 at the C-terminus. | LNFRAPPVIPNVPFLWAWNAPSEFCLGKFDEP LDMSLFSFIGSPRINATGQGVTIFYVDRLGYYP YIDSITGVTVNGGIPQKISLQDHLDKAKKDITF YMPVDNLGMAVIDWEEWRPTWARNWKPKD VYKNRSIELVQQQNVQLSLTEATEKAKQEFEK AGKDFLVETIKLGKLLRPNHLWGYYLFPDCYN HHYKKPGYNGSCFNVEIKRNDDLSWLWNEST ALYPSIYLNTQQSPVAATLYVRNRVREAIRVSK IPDAKSPLPVFAYTRIVFTDQVLKFLSQDELVY TFGETVALGASGIVIWGTWENTRTKESCQAIK EYMDTTLNPYIINVTLAAKMCSQVLCQEQGV CIRKNWNSSDYLHLNPDNFAIQLEKGGKFTVR GKPTLEDLEQFSEKFYCSCYSTLSCKEKADVK DTDAVDVCIADGVC |
| HM31 | 29 | Substitution of 14 amino acids with L342W, S343E, I344N, M345T, S347T, M348K, K349E, L352Q, L353A, L3541, D355K, N356E, E359D, and I361T, truncation before residue L36 at the N-terminus, and truncation after residue D461 at the C-terminus. | LNFRAPPVIPNVPFLWAWNAPSEFCLGKFDEP LDMSLFSFIGSPRINATGQGVTIFYVDRLGYYP YIDSITGVTVNGGIPQKISLQDHLDKAKKDITF |
| | | | YMPVDNLGMAVIDWEEWRPTWARNWKPKD VYKNRSIELVQQQNVQLSLTEATEKAKQEFEK AGKDFLVETIKLGKLLRPNHLWGYYLFPDCYN HHYKKPGYNGSCFNVEIKRNDDLSWLWNEST ALYPSIYLNTQQSPVAATLYVRNRVREAIRVSK IPDAKSPLPVFAYTRIVFTDQVLKFLSQDELVY TFGETVALGASGIVIWGTWENTRTKESCQAIK EYMDTTLNPYIINVTLAAKMCSQVLCQEQGV CIRKNWNSSDYLHLNPDNFAIQLEKGGKFTVR GKPTLEDLEQFSEKFYCSCYSTLSCKEKADVK DTDAVDVCIAD |
| HM32 | 30 | Substitution of 14 amino acids with L342W, S343E, I344N, M345T, S347T, M348K, K349E, L352Q, L353A, L3541, D355K, N356E, E359D, and I361T, truncation before residue L36 at the N-terminus, and truncation after residue C458 at the C-terminus. | LNFRAPPVIPNVPFLWAWNAPSEFCLGKFDEP LDMSLFSFIGSPRINATGQGVTIFYVDRLGYYP YIDSITGVTVNGGIPQKISLQDHLDKAKKDITF YMPVDNLGMAVIDWEEWRPTWARNWKPKD VYKNRSIELVQQQNVQLSLTEATEKAKQEFEK AGKDFLVETIKLGKLLRPNHLWGYYLFPDCYN HHYKKPGYNGSCFNVEIKRNDDLSWLWNEST ALYPSIYLNTQQSPVAATLYVRNRVREAIRVSK IPDAKSPLPVFAYTRIVFTDQVLKFLSQDELVY TFGETVALGASGIVIWGTWENTRTKESCQAIK EYMDTTLNPYIINVTLAAKMCSQVLCQEQGV CIRKNWNSSDYLHLNPDNFAIQLEKGGKFTVR GKPTLEDLEQFSEKFYCSCYSTLSCKEKADVK DTDAVDVC |
| HM33 | 31 | Substitution of 14 amino acids with L342W, S343E, I344N, M345T, S347T, M348K, K349E, L352Q, L353A, L3541, D355K, N356E, E359D, and I361T, truncation before residue L36 at the N-terminus, and truncation after residue V455 at the C-terminus | LNFRAPPVIPNVPFLWAWNAPSEFCLGKFDEP LDMSLFSFIGSPRINATGQGVTIFYVDRLGYYP YIDSITGVTVNGGIPQKISLQDHLDKAKKDITF YMPVDNLGMAVIDWEEWRPTWARNWKPKD VYKNRSIELVQQQNVQLSLTEATEKAKQEFEK AGKDFLVETIKLGKLLRPNHLWGYYLFPDCYN HHYKKPGYNGSCFNVEIKRNDDLSWLWNEST ALYPSIYLNTQQSPVAATLYVRNRVREAIRVSK IPDAKSPLPVFAYTRIVFTDQVLKFLSQDELVY |
| | | | TFGETVALGASGIVIWGTWENTRTKESCQAIK EYMDTTLNPYIINVTLAAKMCSQVLCQEQGV CIRKNWNSSDYLHLNPDNFAIQLEKGGKFTVR GKPTLEDLEQFSEKFYCSCYSTLSCKEKADVK DTDAV |
| HP34 | 32 | Substitution of 15 amino acids with T341S, L342W, S343E, I344N, M345T, S347T, M348K, K349E, L352Q, L353A, L3541, D355K, N356E, E359D, and I361T, truncation before residue F38 at the N-terminus, and truncation after residue K470 at the C-terminus | FRAPPVIPNVPFLWAWNAPSEFCLGKFDEPLD MSLFSFIGSPRINATGQGVTIFYVDRLGYYPYID SITGVTVNGGIPQKISLQDHLDKAKKDITFYMP VDNLGMAVIDWEEWRPTWARNWKPKDVYK NRSIELVQQQNVQLSLTEATEKAKQEFEKAGK DFLVETIKLGKLLRPNHLWGYYLFPDCYNHHY KKPGYNGSCFNVEIKRNDDLSWLWNESTALY PSIYLNTQQSPVAATLYVRNRVREAIRVSKIPD AKSPLPVFAYTRIVFTDQVLKFLSQDELVYTFG ETVALGASGIVIWGSWENTRTKESCQAIKEY MDTTLNPYIINVTLAAKMCSQVLCQEQGVCIR KNWNSSDYLHLNPDNFAIQLEKGGKFTVRGKP TLEDLEQFSEKFYCSCYSTLSCKEKADVKDTD AVDVCIADGVCIDAFLK |
| HM35 | 33 | Substitution of 14 amino acids with L342W, S343E, I344N, M345T, S347T, M348K, K349E, L352Q, L353A, L3541, D355K, N356E, E359D, and I361T, truncation before residue F38 at the N-terminus, and truncation after residue P472 at the C-terminus. | FRAPPVIPNVPFLWAWNAPSEFCLGKFDEPLD MSLFSFIGSPRINATGQGVTIFYVDRLGYYPYID SITGVTVNGGIPQKISLQDHLDKAKKDITFYMP VDNLGMAVIDWEEWRPTWARNWKPKDVYK NRSIELVQQQNVQLSLTEATEKAKQEFEKAGK DFLVETIKLGKLLRPNHLWGYYLFPDCYNHHY KKPGYNGSCFNVEIKRNDDLSWLWNESTALY PSIYLNTQQSPVAATLYVRNRVREAIRVSKIPD AKSPLPVFAYTRIVFTDQVLKFLSQDELVYTFG ETVALGASGIVIWGTWENTRTKESCQAIKEY MDTTLNPYIINVTLAAKMCSQVLCQEQGVCIR KNWNSSDYLHLNPDNFAIQLEKGGKFTVRGKP TLEDLEQFSEKFYCSCYSTLSCKEKADVKDTD AVDVCIADGVCIDAFLKPP |
| HM36 | 34 | Substitution of 14 amino acids with L342W, S343E, I344N, M345T, S347T, M348K, K349E, L352Q, L353A, L3541, D355K, N356E, E359D, and I361T, truncation before residue F38 at the N-terminus, and truncation after residue M473 at the C-terminus. | FRAPPVIPNVPFLWAWNAPSEFCLGKFDEPLD MSLFSFIGSPRINATGQGVTIFYVDRLGYYPYID SITGVTVNGGIPQKISLQDHLDKAKKDITFYMP VDNLGMAVIDWEEWRPTWARNWKPKDVYK NRSIELVQQQNVQLSLTEATEKAKQEFEKAGK DFLVETIKLGKLLRPNHLWGYYLFPDCYNHHY KKPGYNGSCFNVEIKRNDDLSWLWNESTALY PSIYLNTQQSPVAATLYVRNRVREAIRVSKIPD AKSPLPVFAYTRIVFTDQVLKFLSQDELVYTFG ETVALGASGIVIWGTWENTRTKESCQAIKEY MDTTLNPYIINVTLAAKMCSQVLCQEQGVCIR KNWNSSDYLHLNPDNFAIQLEKGGKFTVRGKP TLEDLEQFSEKFYCSCYSTLSCKEKADVKDTD AVDVCIADGVCIDAFLKPPM |
| HM37 | 35 | Substitution of 14 amino acids with L342W, S343E, I344N, M345T, S347T, M348K, K349E, L352Q, L353A, L3541, D355K, N356E, E359D, and I361T, truncation before residue F38 at the N-terminus, and truncation after residue E474 at the C-terminus. | FRAPPVIPNVPFLWAWNAPSEFCLGKFDEPLD MSLFSFIGSPRINATGQGVTIFYVDRLGYYPYID SITGVTVNGGIPQKISLQDHLDKAKKDITFYMP VDNLGMAVIDWEEWRPTWARNWKPKDVYK NRSIELVQQQNVQLSLTEATEKAKQEFEKAGK DFLVETIKLGKLLRPNHLWGYYLFPDCYNHHY KKPGYNGSCFNVEIKRNDDLSWLWNESTALY PSIYLNTQQSPVAATLYVRNRVREAIRVSKIPD AKSPLPVFAYTRIVFTDQVLKFLSQDELVYTFG ETVALGASGIVIWGTWENTRTKESCQAIKEY MDTTLNPYIINVTLAAKMCSQVLCQEQGVCIR KNWNSSDYLHLNPDNFAIQLEKGGKFTVRGKP TLEDLEQFSEKFYCSCYSTLSCKEKADVKDTD AVDVCIADGVCIDAFLKPPME |
| HM38 | 36 | Substitution of 14 amino acids with L342W, S343E, I344N, M345T, S347T, M348K, K349E, L352Q, L353A, L3541, D355K, N356E, E359D, and I361T, truncation before residue F38 at the N-terminus, and truncation after residue T475 at the C-terminus. | FRAPPVIPNVPFLWAWNAPSEFCLGKFDEPLD MSLFSFIGSPRINATGQGVTIFYVDRLGYYPYID SITGVTVNGGIPQKISLQDHLDKAKKDITFYMP VDNLGMAVIDWEEWRPTWARNWKPKDVYK NRSIELVQQQNVQLSLTEATEKAKQEFEKAGK DFLVETIKLGKLLRPNHLWGYYLFPDCYNHHY |
| | | | KKPGYNGSCFNVEIKRNDDLSWLWNESTALY PSIYLNTQQSPVAATLYVRNRVREAIRVSKIPD AKSPLPVFAYTRIVFTDQVLKFLSQDELVYTFG ETVALGASGIVIWGTWENTRTKESCQAIKEY MDTTLNPYIINVTLAAKMCSQVLCQEQGVCIR KNWNSSDYLHLNPDNFAIQLEKGGKFTVRGKP TLEDLEQFSEKFYCSCYSTLSCKEKADVKDTD AVDVCIADGVCIDAFLKPPMET |
| HM39 | 37 | Substitution of 14 amino acids with L342W, S343E, I344N, M345T, S347T, M348K, K349E, L352Q, L353A, L3541, D355K, N356E, E359D, and I361T, truncation before residue F38 at the N-terminus, and truncation after residue E476 at the C-terminus. | FRAPPVIPNVPFLWAWNAPSEFCLGKFDEPLD MSLFSFIGSPRINATGQGVTIFYVDRLGYYPYID SITGVTVNGGIPQKISLQDHLDKAKKDITFYMP VDNLGMAVIDWEEWRPTWARNWKPKDVYK NRSIELVQQQNVQLSLTEATEKAKQEFEKAGK DFLVETIKLGKLLRPNHLWGYYLFPDCYNHHY KKPGYNGSCFNVEIKRNDDLSWLWNESTALY PSIYLNTQQSPVAATLYVRNRVREAIRVSKIPD AKSPLPVFAYTRIVFTDQVLKFLSQDELVYTFG ETVALGASGIVIWGTWENTRTKESCQAIKEY MDTTLNPYIINVTLAAKMCSQVLCQEQGVCIR KNWNSSDYLHLNPDNFAIQLEKGGKFTVRGKP TLEDLEQFSEKFYCSCYSTLSCKEKADVKDTD AVDVCIADGVCIDAFLKPPMETE |
| HM40 | 38 | Substitution of 11 amino acids with M345T, S347T, M348K, K349E, L352Q, L353A, L3541, D355K, N356E, E359D, and I361T, and truncation before residue N37 at the N-terminus. | NFRAPPVIPNVPFLWAWNAPSEFCLGKFDEPL DMSLFSFIGSPRINATGQGVTIFYVDRLGYYPYI DSITGVTVNGGIPQKISLQDHLDKAKKDITFYM PVDNLGMAVIDWEEWRPTWARNWKPKDVYK NRSIELVQQQNVQLSLTEATEKAKQEFEKAGK DFLVETIKLGKLLRPNHLWGYYLFPDCYNHHY KKPGYNGSCFNVEIKRNDDLSWLWNESTALY PSIYLNTQQSPVAATLYVRNRVREAIRVSKIPD AKSPLPVFAYTRIVFTDQVLKFLSQDELVYTFG ETVALGASGIVIWGTLSITRTKESCQAIKEYM DTTLNPYIINVTLAAKMCSQVLCQEQGVCIRK NWNSSDYLHLNPDNFAIQLEKGGKFTVRGKPT |
| | | | LEDLEQFSEKFYCSCYSTLSCKEKADVKDTDA VDVCIADGVCIDAFLKPPMETEEPQIFYNASPS TLS |
| HM41 | 39 | Substitution of 11 amino acids with M345T, S347T, M348K, K349E, L352Q, L353A, L3541, D355K, N356E, E359D, and I361T, and truncation before residue R39 at the N-terminus. | RAPPVIPNVPFLWAWNAPSEFCLGKFDEPLDM SLFSFIGSPRINATGQGVTIFYVDRLGYYPYIDSI TGVTVNGGIPQKISLQDHLDKAKKDITFYMPV DNLGMAVIDWEEWRPTWARNWKPKDVYKNR SIELVQQQNVQLSLTEATEKAKQEFEKAGKDF LVETIKLGKLLRPNHLWGYYLFPDCYNHHYK KPGYNGSCFNVEIKRNDDLSWLWNESTALYPS IYLNTQQSPVAATLYVRNRVREAIRVSKIPDAK SPLPVFAYTRIVFTDQVLKFLSQDELVYTFGET VALGASGIVIWGTLSITRTKESCQAIKEYMDT TLNPYIINVTLAAKMCSQVLCQEQGVCIRKNW NSSDYLHLNPDNFAIQLEKGGKFTVRGKPTLE DLEQFSEKFYCSCYSTLSCKEKADVKDTDAVD VCIADGVCIDAFLKPPMETEEPQIFYNASPSTLS |
| HM42 | 40 | Substitution of 11 amino acids with M345T, S347T, M348K, K349E, L352Q, L353A, L3541, D355K, N356E, E359D, and I361T, and truncation before residue P41 at the N-terminus. | PPVIPNVPFLWAWNAPSEFCLGKFDEPLDMSL FSFIGSPRINATGQGVTIFYVDRLGYYPYIDSIT GVTVNGGIPQKISLQDHLDKAKKDITFYMPVD NLGMAVIDWEEWRPTWARNWKPKDVYKNRS IELVQQQNVQLSLTEATEKAKQEFEKAGKDFL VETIKLGKLLRPNHLWGYYLFPDCYNHHYKK PGYNGSCFNVEIKRNDDLSWLWNESTALYPSI YLNTQQSPVAATLYVRNRVREAIRVSKIPDAK SPLPVFAYTRIVFTDQVLKFLSQDELVYTFGET VALGASGIVIWGTLSITRTKESCQAIKEYMDT TLNPYIINVTLAAKMCSQVLCQEQGVCIRKNW NSSDYLHLNPDNFAIQLEKGGKFTVRGKPTLE DLEQFSEKFYCSCYSTLSCKEKADVKDTDAVD VCIADGVCIDAFLKPPMETEEPQIFYNASPSTLS |
| HM43 | 41 | Substitution of 14 amino acids with L342W, S343E, I344N, M345T, S347T, M348K, K349E, L352Q, L353A, L3541, D355K, N356E, E359D, and I361T, truncation before residue F38 at the N-terminus, and truncation after residue 1465 at the C-terminus. | FRAPPVIPNVPFLWAWNAPSEFCLGKFDEPLD MSLFSFIGSPRINATGQGVTIFYVDRLGYYPYID SITGVTVNGGIPQKISLQDHLDKAKKDITFYMP |
| | | | VDNLGMAVIDWEEWRPTWARNWKPKDVYK NRSIELVQQQNVQLSLTEATEKAKQEFEKAGK DFLVETIKLGKLLRPNHLWGYYLFPDCYNHHY KKPGYNGSCFNVEIKRNDDLSWLWNESTALY PSIYLNTQQSPVAATLYVRNRVREAIRVSKIPD AKSPLPVFAYTRIVFTDQVLKFLSQDELVYTFG ETVALGASGIVIWGSWENTRTKESCQAIKEY MDTTLNPYIINVTLAAKMCSQVLCQEQGVCIR KNWNSSDYLHLNPDNFAIQLEKGGKFTVRGKP TLEDLEQFSEKFYCSCYSTLSCKEKADVKDTD AVDVCIADGVCI |
| HM44 | 42 | Substitution of 14 amino acids with L342W, S343E, I344N, M345T, S347T, M348K, K349E, L352Q, L353A, L3541, D355K, N356E, E359D, and I361T, truncation before residue F38 at the N-terminus, and truncation after residue D466 at the C-terminus. | FRAPPVIPNVPFLWAWNAPSEFCLGKFDEPLD MSLFSFIGSPRINATGQGVTIFYVDRLGYYPYID SITGVTVNGGIPQKISLQDHLDKAKKDITFYMP VDNLGMAVIDWEEWRPTWARNWKPKDVYK NRSIELVQQQNVQLSLTEATEKAKQEFEKAGK DFLVETIKLGKLLRPNHLWGYYLFPDCYNHHY KKPGYNGSCFNVEIKRNDDLSWLWNESTALY PSIYLNTQQSPVAATLYVRNRVREAIRVSKIPD AKSPLPVFAYTRIVFTDQVLKFLSQDELVYTFG ETVALGASGIVIWGSWENTRTKESCQAIKEY MDTTLNPYIINVTLAAKMCSQVLCQEQGVCIR KNWNSSDYLHLNPDNFAIQLEKGGKFTVRGKP TLEDLEQFSEKFYCSCYSTLSCKEKADVKDTD AVDVCIADGVCID |
| HM45 | 43 | Substitution of 14 amino acids with L342W, S343E, I344N, M345T, S347T, M348K, K349E, L352Q, L353A, L3541, D355K, N356E, E359D, and I361T, truncation before residue F38 at the N-terminus, and truncation after residue A467 at the C-terminus. | FRAPPVIPNVPFLWAWNAPSEFCLGKFDEPLD MSLFSFIGSPRINATGQGVTIFYVDRLGYYPYID SITGVTVNGGIPQKISLQDHLDKAKKDITFYMP VDNLGMAVIDWEEWRPTWARNWKPKDVYK NRSIELVQQQNVQLSLTEATEKAKQEFEKAGK DFLVETIKLGKLLRPNHLWGYYLFPDCYNHHY KKPGYNGSCFNVEIKRNDDLSWLWNESTALY PSIYLNTQQSPVAATLYVRNRVREAIRVSKIPD AKSPLPVFAYTRIVFTDQVLKFLSQDELVYTFG |
| | | | ETVALGASGIVIWGSWENTRTKESCQAIKEY MDTTLNPYIINVTLAAKMCSQVLCQEQGVCIR KNWNSSDYLHLNPDNFAIQLEKGGKFTVRGKP TLEDLEQFSEKFYCSCYSTLSCKEKADVKDTD AVDVCIADGVCIDA |
| HP46 | 44 | Substitution of 15 amino acids with T341S, L342W, S343E, I344N, M345T, S347T, M348K, K349E, L352Q, L353A, L3541, D355K, N356E, E359D, and 1361T, truncation before residue F38 at the N-terminus, and truncation after residue F468 at the C-terminus. | FRAPPVIPNVPFLWAWNAPSEFCLGKFDEPLD MSLFSFIGSPRINATGQGVTIFYVDRLGYYPYID SITGVTVNGGIPQKISLQDHLDKAKKDITFYMP VDNLGMAVIDWEEWRPTWARNWKPKDVYK NRSIELVQQQNVQLSLTEATEKAKQEFEKAGK DFLVETIKLGKLLRPNHLWGYYLFPDCYNHHY KKPGYNGSCFNVEIKRNDDLSWLWNESTALY PSIYLNTQQSPVAATLYVRNRVREAIRVSKIPD AKSPLPVFAYTRIVFTDQVLKFLSQDELVYTFG ETVALGASGIVIWGSWENTRTKESCQAIKEY MDTTLNPYIINVTLAAKMCSQVLCQEQGVCIR KNWNSSDYLHLNPDNFAIQLEKGGKFTVRGKP TLEDLEQFSEKFYCSCYSTLSCKEKADVKDTD AVDVCIADGVCIDAF |
| HM47 | 45 | Substitution of 14 amino acids with L342W, S343E, I344N, M345T, S347T, M348K, K349E, L352Q, L353A, L3541, D355K, N356E, E359D, and I361T, truncation before residue F38 at the N-terminus, and truncation after residue P478 at the C-terminus. | FRAPPVIPNVPFLWAWNAPSEFCLGKFDEPLD MSLFSFIGSPRINATGQGVTIFYVDRLGYYPYID SITGVTVNGGIPQKISLQDHLDKAKKDITFYMP VDNLGMAVIDWEEWRPTWARNWKPKDVYK NRSIELVQQQNVQLSLTEATEKAKQEFEKAGK DFLVETIKLGKLLRPNHLWGYYLFPDCYNHHY KKPGYNGSCFNVEIKRNDDLSWLWNESTALY PSIYLNTQQSPVAATLYVRNRVREAIRVSKIPD AKSPLPVFAYTRIVFTDQVLKFLSQDELVYTFG ETVALGASGIVIWGTWENTRTKESCQAIKEY MDTTLNPYIINVTLAAKMCSQVLCQEQGVCIR KNWNSSDYLHLNPDNFAIQLEKGGKFTVRGKP TLEDLEQFSEKFYCSCYSTLSCKEKADVKDTD AVDVCIADGVCIDAFLKPPMETEEP |
| HM48 | 46 | Substitution of 14 amino acids with L342W, S343E, I344N, M345T, S347T, M348K, K349E, L352Q, L353A, L3541, D355K, N356E, E359D, and I361T, truncation before residue F38 at the N-terminus, and truncation after residue 1480 at the C-terminus. | FRAPPVIPNVPFLWAWNAPSEFCLGKFDEPLD MSLFSFIGSPRINATGQGVTIFYVDRLGYYPYID SITGVTVNGGIPQKISLQDHLDKAKKDITFYMP VDNLGMAVIDWEEWRPTWARNWKPKDVYK NRSIELVQQQNVQLSLTEATEKAKQEFEKAGK DFLVETIKLGKLLRPNHLWGYYLFPDCYNHHY KKPGYNGSCFNVEIKRNDDLSWLWNESTALY PSIYLNTQQSPVAATLYVRNRVREAIRVSKIPD AKSPLPVFAYTRIVFTDQVLKFLSQDELVYTFG ETVALGASGIVIWGTWENTRTKESCQAIKEY MDTTLNPYIINVTLAAKMCSQVLCQEQGVCIR KNWNSSDYLHLNPDNFAIQLEKGGKFTVRGKP TLEDLEQFSEKFYCSCYSTLSCKEKADVKDTD AVDVCIADGVCIDAFLKPPMETEEPQI |
| HM49 | 47 | Substitution of 14 amino acids with L342W, S343E, I344N, M345T, S347T, M348K, K349E, L352Q, L353A, L3541, D355K, N356E, E359D, and I361T, truncation before residue F38 at the N-terminus, and truncation after residue Y482 at the C-terminus. | FRAPPVIPNVPFLWAWNAPSEFCLGKFDEPLD MSLFSFIGSPRINATGQGVTIFYVDRLGYYPYID SITGVTVNGGIPQKISLQDHLDKAKKDITFYMP VDNLGMAVIDWEEWRPTWARNWKPKDVYK NRSIELVQQQNVQLSLTEATEKAKQEFEKAGK DFLVETIKLGKLLRPNHLWGYYLFPDCYNHHY KKPGYNGSCFNVEIKRNDDLSWLWNESTALY PSIYLNTQQSPVAATLYVRNRVREAIRVSKIPD AKSPLPVFAYTRIVFTDQVLKFLSQDELVYTFG ETVALGASGIVIWGTWENTRTKESCQAIKEY MDTTLNPYIINVTLAAKMCSQVLCQEQGVCIR KNWNSSDYLHLNPDNFAIQLEKGGKFTVRGKP TLEDLEQFSEKFYCSCYSTLSCKEKADVKDTD AVDVCIADGVCIDAFLKPPMETEEPQIFY |
| HM50 | 48 | Substitution of 14 amino acids with L342W, S343E, I344N, M345T, S347T, M348K, K349E, L352Q, L353A, L3541, D355K, N356E, E359D, and I361T, truncation before residue F38 at the N-terminus, and truncation after residue A484 at the C-terminus. | FRAPPVIPNVPFLWAWNAPSEFCLGKFDEPLD MSLFSFIGSPRINATGQGVTIFYVDRLGYYPYID SITGVTVNGGIPQKISLQDHLDKAKKDITFYMP VDNLGMAVIDWEEWRPTWARNWKPKDVYK NRSIELVQQQNVQLSLTEATEKAKQEFEKAGK DFLVETIKLGKLLRPNHLWGYYLFPDCYNHHY |
| | | | KKPGYNGSCFNVEIKRNDDLSWLWNESTALY PSIYLNTQQSPVAATLYVRNRVREAIRVSKIPD AKSPLPVFAYTRIVFTDQVLKFLSQDELVYTFG ETVALGASGIVIWGTWENTRTKESCQAIKEY MDTTLNPYIINVTLAAKMCSQVLCQEQGVCIR KNWNSSDYLHLNPDNFAIQLEKGGKFTVRGKP TLEDLEQFSEKFYCSCYSTLSCKEKADVKDTD AVDVCIADGVCIDAFLKPPMETEEPQIFYNA |
| HM51 | 49 | Substitution of 14 amino acids with L342W, S343E, I344N, M345T, S347T, M348K, K349E, L352Q, L353A, L3541, D355K, N356E, E359D, and I361T, truncation before residue F38 at the N-terminus, and truncation after residue P486 at the C-terminus. | FRAPPVIPNVPFLWAWNAPSEFCLGKFDEPLD MSLFSFIGSPRINATGQGVTIFYVDRLGYYPYID SITGVTVNGGIPQKISLQDHLDKAKKDITFYMP VDNLGMAVIDWEEWRPTWARNWKPKDVYK NRSIELVQQQNVQLSLTEATEKAKQEFEKAGK DFLVETIKLGKLLRPNHLWGYYLFPDCYNHHY KKPGYNGSCFNVEIKRNDDLSWLWNESTALY PSIYLNTQQSPVAATLYVRNRVREAIRVSKIPD AKSPLPVFAYTRIVFTDQVLKFLSQDELVYTFG ETVALGASGIVIWGTWENTRTKESCQAIKEY MDTTLNPYIINVTLAAKMCSQVLCQEQGVCIR KNWNSSDYLHLNPDNFAIQLEKGGKFTVRGKP TLEDLEQFSEKFYCSCYSTLSCKEKADVKDTD AVDVCIADGVCIDAFLKPPMETEEPQIFYNASP |
| HM52 | 50 | Substitution of 14 amino acids with L342W, S343E, I344N, M345T, S347T, M348K, K349E, L352Q, L353A, L3541, D355K, N356E, E359D, and I361T, truncation before residue F38 at the N-terminus, and truncation after residue T488 at the C-terminus. | FRAPPVIPNVPFLWAWNAPSEFCLGKFDEPLD MSLFSFIGSPRINATGQGVTIFYVDRLGYYPYID SITGVTVNGGIPQKISLQDHLDKAKKDITFYMP VDNLGMAVIDWEEWRPTWARNWKPKDVYK NRSIELVQQQNVQLSLTEATEKAKQEFEKAGK DFLVETIKLGKLLRPNHLWGYYLFPDCYNHHY KKPGYNGSCFNVEIKRNDDLSWLWNESTALY PSIYLNTQQSPVAATLYVRNRVREAIRVSKIPD AKSPLPVFAYTRIVFTDQVLKFLSQDELVYTFG ETVALGASGIVIWGTWENTRTKESCQAIKEY MDTTLNPYIINVTLAAKMCSQVLCQEQGVCIR KNWNSSDYLHLNPDNFAIQLEKGGKFTVRGKP |
| | | | TLEDLEQFSEKFYCSCYSTLSCKEKADVKDTD AVDVCIADGVCIDAFLKPPMETEEPQIFYNASP ST |

Meanwhile, the previous research showed that the enzymatic activity of the wild-type human PH20 changes depending on the position of truncation of the amino acid residue located at the C-terminus. However, according to the present invention, human PH20 variants that are more stable than wild-type human PH20 were produced by substituting the specific alpha helix of the secondary structure of human PH20 with the alpha helix of another human hyaluronidase and these variants are different from wild-type PH20 in terms of the interaction between the substituted alpha-helical domain and another secondary structures of PH20 and thus have enzymatic activity of a predetermined level or higher, regardless of the position of the C-terminal truncation.

In addition, in the present invention, the expression of recombinant human PH20 or a variant thereof was improved using a signal peptide of another protein highly expressed in animal cells, rather than a signal peptide unique to human PH20 or a variant thereof.

Accordingly, in another aspect, the PH20 variant contained in the pharmaceutical composition according to the present invention includes a signal peptide derived from human hyaluronidase-1 (Hyal1), human growth hormone or human serum albumin instead of the M1 to T35 wild-type PH20 signal peptide, and preferably includes a signal peptide derived from albumin, preferably a signal peptide derived from human growth hormone having an amino acid sequence of MATGSRTSLLLAFGLLCLPWLQEGSA represented by SEQ ID NO: 2, a signal peptide derived from human serum albumin having an amino acid sequence of MKWVTFISLLFLFSSAYS represented by SEQ ID NO: 3, or a signal peptide derived from human Hyal1 having the amino acid sequence of MAAHLLPICALFLTLLDMAQG represented by SEQ ID NO: 4 as shown in Table 5, but is not limited thereto.

**Table 5. Amino acid sequence of signal peptide of human growth hormone, human serum albumin, or human Hyal1**

| Origin of signal peptide | | Amino acid sequence | SEQ ID NO. |
|---|---|---|---|
| Human hormone | growth | MATGSRTSLLLAFGLLCLPWLQEGSA | 2 |
| Human albumin | serum | MKWVTFISLLFLFSSAYS | 3 |
| Human Hyal1 | | MAAHLLPICALFLTLLDMAQG | 4 |

Among the PH20 variants contained in the pharmaceutical composition according to the present invention, the variant with 6xHis-tag attached to the C-terminus thereof was referred to as "HM", and the variant having no 6xHis-tag attached to the C-terminus thereof was referred to as "HP". In addition, the mature wild-type PH20 (L36-S490) with a 6xHis-tag attached to the C-terminus thereof was referred to as "WT", and the mature wild-type PH20 (L36-Y482) with no 6xHis-tag and the C-terminus truncated after Y482 was referred to as "HW2".

HP46 (SEQ ID NO: 44) is a variant of human PH20 formed by modeling the protein structure with Hyal1 (PDB ID: 2PE4) (Chao et al., 2007), a human hyaluronidase, the tertiary structure of which was known, and substituting the amino acid sequence of alpha helix 8 of human PH20 and the linker region between alpha helix 7 and alpha helix 8 with the amino acid sequence of Hyal1, truncating the N-terminus at F38 and truncating the C-terminus after F468.

The pharmaceutical composition according to the present invention contains at least 50 units/mL, preferably 100 to 20,000 units/mL, more preferably about 150 to 18,000 units/mL, still more preferably 1,000 to 16,000 units/mL, most preferably 1,500 to 12,000 units/mL of PH20 or PH20 variants.

Examples of the drug contained in the pharmaceutical composition according to the present invention include, but are not limited to, protein drugs, antibody drugs, small molecules, aptamers, RNAi, antisense, and cell therapeutic agents such as CAR (chimeric antigen receptor)-T and CAR-NK (natural killers), and include commercially available drugs, and drugs currently in clinical and developmental stages.

Preferably, the drug is a protein drug or an antibody drug.

The "protein drug" contained in the pharmaceutical composition according to the present invention refers to a drug that is composed of amino acids and thus has a therapeutic or prophylactic effect for diseases through the activity of proteins, which means a pharmaceutical product containing proteins other than antibody drugs, and is selected from the group consisting of cytokines, therapeutic enzymes, hormones, soluble receptors and fusion proteins thereof, insulin or analogues thereof, bone morphogenetic proteins (BMP), erythropoietin (EPO) and serum-derived proteins, but is not limited thereto.

The cytokines contained in the pharmaceutical composition according to the present invention may be selected from the group consisting of interferons, interleukins, colony stimulating factors (CSF), tumor necrosis factors (TNF), and tissue growth factors (TGF), but is not limited thereto, and examples of the therapeutic enzymes include beta-glucocerebrosidase and agalsidase beta, but are not limited thereto.

Examples of cytokines and similar products include oprelvekin, peginterferon Beta-1a, pegilodecakin, rezpegaldesleukin, SAR 444245, GO-203-2c, dekavil, nemvaleukin alfa, efavaleukin alfa, efineptakin alfa, onfekafusp alfa, bifikafusp alfa, lerodalcibep and the like, but are not limited thereto.

The soluble receptor contained in the pharmaceutical composition according to the present invention refers to an extracellular domain of the receptor and a fusion protein thereof refers to a protein in which the Fc region of an antibody is fused to a soluble receptor. Examples of the soluble receptor include, but are not limited to, a watersoluble form of a receptor to which a disease-related ligand binds, a form in which an Fc region is fused to a TNF-α soluble receptor (e.g., a product with an ingredient name called "Etanercept" and a similar form), a form in which a Fc region is fused to a VEGF soluble receptor (product with an ingredient name called "Aflibercept", "Conbercept" or "OPT-302" and a similar form), a form in which a Fc region is fused to CTLA-4 (for example, product with an ingredient name called "Abatacept" or "Belatacept" and a similar form), a form in which a Fc region is fused to an interleukin soluble receptor (for example, a product with an ingredient name called "Rilonacept" or "Inbakicept" and a similar form), a form in which an Fc region is fused to a LFA3 soluble receptor (e.g., products with an ingredient name "Alefacept" and similar forms), a form in which an Fc region is fused to signal regulatory protein alpha (SIRPalpha) that interacts with CD47 (e.g., products with ingredient names called "Evorpacept", "Ontorpacept", or "TTI-622" and similar forms), products with ingredient names called "Sotatercept", "Dalantercept", "Acazicolcept", or "Dazodalibep", and similar forms.

The hormones contained in the pharmaceutical composition according to the present invention refer to hormones or analogues thereof injected from an external source for the treatment or prevention of diseases caused by hormone deficiency or the like, and examples of the hormones include human growth hormones, estrogen, progesterone and the like, but are not limited thereto.

The serum-derived proteins contained in the pharmaceutical composition according to the present invention are proteins present in serum, encompass both proteins extracted from serum and proteins produced recombinantly, and include fibrinogen, von Willebrand factor, albumin, thrombin, FIT (factor II) , FV (factor V), FVII (factor VII), FVIII (factor VIII), FIX (factor IX), FX (factor X) and FXI (factor XI), and the like, but are not limited thereto.

The antibody drug contained in the pharmaceutical composition according to the present invention may be a monoclonal antibody drug or a polyclonal antibody drug.

The monoclonal antibody drug according to the present invention refers to a protein including a monoclonal antibody and a monoclonal antibody fragment capable of specifically binding to an antigen associated with a specific disease. A monoclonal antibody also includes a bispecific antibody and a protein including a monoclonal antibody or a fragment thereof includes an antibody-drug conjugate (ADC).

Examples of antigens associated with specific diseases include 4-1BB, 5T4, integrin, activin, amyloid beta, angiopoietin 1 or 2, angiopoietin analogue 3, B cell maturation antigen (BCMA), B-cell activating factors (BAFF), B7-H3, complement 5, CCR4, CCR5, CCL11, CD2, CD3, CD4, CD6, CD11a, CD16A, CD19, CD20, CD22, CD25, CD27, CD28, CD30, CD32B, CD33, CD38, CD40, CD45, CD46, CD47, CD52, CD56, CD62, CD70, CD73, CD74, CD79b, CD80, CD105, CD123, CD154, CD166, CD262, CD278, CD319, CD326, carcinoembryonic antigen (CEA), CGRP, claudin-18, c-Met, CSF-1, CSF-1 receptors, CTLA4, DLL3, EGF receptors, hemophilia factors, Fc receptors, FGF23, folate receptors, GD2, glucocorticoid-induced TNF receptors (GITR), glypican 3, GM-CSF, HER2, HER3, hepatocyte growth factors (HGF), interferon receptors, interferon gamma, IgE, IGF-1 receptors, interleukin 1, interleukin 2 receptors, interleukin 4, interleukin 4 receptors, interleukin 5, interleukin 5 receptors, interleukin 6, interleukin 6 receptors, interleukin 8, interleukin 12/23, interleukin 13, interleukin 17A, interleukin 17 receptor A, interleukin 23, interleukin 31 receptors, interleukin 36 receptors, lymphocyte-activation gene 3 (LAG3), Lysyl oxidase homolog 2 (LOXL2), mesothelin, mucin-1, mucin-16, nectin 4, nerve growth factors (NGF), OX40, proprotein convertase subtilisin/kexin type 9 (PCSK9), PD-1, PD-L1, phospholipase C, RANKL (receptor activator of nuclear factors kappa B ligand), tyrosine-protein kinase transmembrane receptor (ROR1), sialic acid binding ig-like lectin 15 (Siglec-15), transforming growth factor beta (TGFβ), TIGIT (T-cell immunoreceptor with immunoglobulin and ITIM domain), T-cell immunoglobulin and mucin-domain containing-3 (Tim-3), tissue factors, tissue factor pathway inhibitors (TFPI), TORP-2, tumor necrosis factors (TNF), thymic stromal lymphopoietin (TSLB), large colony stimulating factor 1 receptor (CSF1R), vascular endothelial growth factor (VEGF), VEGF receptors and von Willebrand Factor (vWF), but are not limited thereto.

In addition, examples of proteins containing monoclonal antibodies and monoclonal antibody fragments against antigens related to specific diseases, bi/multispecific antibodies, and antibody-drug conjugates will be given as follows, but are not limited thereto:
Examples of proteins, including monoclonal antibodies and monoclonal antibody fragments include, but are not limited to, ADG 106, EU101, LVGN6051, urelumab, utomilumab, bebtelovimab, aducanumab, bapinezumab, crenezumab, donanemab, gantenerumab, lecanemab, solanezumab, nesvacumab, evinacumab, enoblituzumab, omburtamab, belimumab, ianalumab, tabalumab, bertilimumab, mogamulizumab, leronlimab, siplizumab, foralumab, muromonab-CD3, otelixizumab, teplizumab, ibalizumab, tregalizumab, zanolimumab, itolizumab, efalizumab, inebilizumab, tafasitamab, tositumomab, ocrelizumab, ofatumumab, rituximab, ublituximab, veltuzumab, epratuzumab, basiliximab, daclizumab, varlilumab, lulizumab pegol, iratumumab, BI-1206, lintuzumab, daratumumab, felzartamab, GEN3014, isatuximab, mezagitamab, CDX-1140, bleselumab, dacetuzumab, iscalimab, lucatumumab, mitazalimab, SEA-CD40, sotigalimab, SAR441344, tegoprubart, dapirolizumab pegol, I-131-apamistamab, AO-176, ligufalimab, magrolimab, alemtuzumab, crizanlizumab, inclacumab, cusatuzumab, oleclumab, milatuzumab, galiximab, carotuximab, adecatumumab, eptinezumab, erenumab, fremanezumab, galcanezumab, TST001, ZL-1211, zolbetuximab, onartuzumab, eculizumab, pozelimab, ravulizumab, lacnotuzumab, PD-0360324, AMB-051, axatilimab, cabiralizumab, emactuzumab, BA3071, ipilimumab, quavonlimab, tremelimumab, zalifrelimab, cetuximab, depatuxizumab, futuximab, imgatuzumab, matuzumab, modotuximab, necitumumab, nimotuzumab, panitumumab, tomuzotuximab, zalutumumab, MK-2060, batoclimab, nipocalimab, rozanolixizumab, burosumab, farletuzumab, dinutuximab, dinutuximab beta, naxitamab, BMS-986156, ragifilimab, gimsilumab, lenzilumab, mavrilimumab, namilumab, otilimab, plonmarlimab, codrituzumab, margetuximab, pertuzumab, trastuzumab, HMBD-001, patritumab, seribantumab, duligotuzumab, ficlatuzumab, rilotumumab, alomfilimab, anifrolumab, emapalumab, FB825, ligelizumab, omalizumab, cixutumumab, dalotuzumab, figitumumab, ganitumab, teprotumumab, bermekimab, canakinumab, gevokizumab, briakinumab, ustekinumab, anrukinzumab, cendakimab, lebrikizumab, tralokinumab, brodalumab, bimekizumab, ixekizumab, secukinumab, brazikumab, guselkumab, mirikizumab, risankizumab, tildrakizumab, nemolizumab, imsidolimab, spesolimab, pascolizumab, CBP201, dupilumab, depemokimab, mepolizumab, reslizumab, benralizumab, clazakizumab, olokizumab, siltuximab, sirukumab, ziltivekimab, levilimab, sarilumab, satralizumab, tocilizumab, HuMax-IL8, abituzumab, favezelimab, fianlimab, GSK2831781, ieramilimab, INCAGN02385, relatlimab, simtuzumab, abagovomab, oregovomab, tanezumab, BMS-986178, GSK3174998, INCAGN01949, ivuxolimab, rocatinlimab, tavolimab, telazorlimab, vonlerolizumab, alirocumab, bococizumab, ebronucimab, evolocumab, frovocimab, ongericimab, tafolecimab, dostarlimab, balstilimab, camrelizumab, cemiplimab, geptanolimab, MEDI0680, nivolumab, pembrolizumab, penpulimab, pidilizumab, prolgolimab, retifanlimab, sasanlimab, serplulimab, sintilimab, spartalizumab, tislelizumab, toripalimab, ezabenlimab, zimberelimab, atezolizumab, avelumab, cosibelimab, sugemalimab, durvalumab, IMC-001, envafolimab, suvratoxumab, denosumab, zilovertamab, NC318, elotuzumab, NIS793, BMS-986207, domvanalimab, EOS-448, etigilimab, ociperlimab, tiragolumab, vibostolimab, surzebiclimab, cobolimab, sabatolimab, TQB2618, concizumab, marstacimab, adalimumab, golimumab, infliximab, certolizumab pegol, conatumumab, tigatuzumab, tezepelumab, gatipotuzumab, cabiralizumab, bevacizumab, brolucizumab, ranibizumab, olinvacimab, icrucumab, ramucirumab, caplacizumab, abrilumab, etrolizumab, vedolizumab, intetumumab, and natalizumab.

Examples of the bi/multispecific antibodies and antibody-like proteins include, but are not limited to, DSP107, RO7122290, cinrebafusp alfa, GEN1042, rozibafusp alfa, GEN1044, obrindatamab, GEN1047, elranatamab, linvoseltamab, teclistamab, epcoritamab, glofitamab, mosunetuzumab, odronextamab, flotetuzumab, vibecotamab, catumaxomab, cibisatamab, TAK-186, talquetamab, ubamatamab, NVG-111, emfizatamab, HPN536, AFM13, blinatumomab, ISB1442, CPO107, AFM24, amivantamab, MCLA-129, SI-B001, emicizumab, Mim8, zenocutuzumab, zanidatamab, tibulizumab, GI-101, vobarilizumab, REGN5668, HX009, cadonilimab, vudalimab, EMB-02, RO7247669, tebotelimab, IBI318, AZD2936, AZD7789, RO7121661, ivonescimab, IBI322, ES101, GEN1046, PRS-344, erfonrilimab, FS118, bintrafusp alfa, HB0036, HLX301, NM21-1480, ozoralizumab, BI836880, faricimab, vanucizumab, navicixizumab, and IBI302.

Examples of the antibody-drug conjugates include, but are not limited to, naptumomab estafenatox, belantamab mafodotin, DS-7300, MGC018, pivekimab sunirine, praluzatamab ravtansine, coltuximab ravtansine, denintuzumab mafodotin, loncastuximab tesirine, ibritumomab tiuxetan, inotuzumab ozogamicin, epratuzumab-cys-tesirine, moxetumomab pasudotox, brentuximab vedotin, gemtuzumab ozogamicin, vadastuximab talirine, STI-6129, FOR46, lorvotuzumab mertansine, polatuzumab vedotin, tusamitamab ravtansine, telisotuzumab vedotin, rovalpituzumab tesirine, depatuxizumab mafodotin, farletuzumab ecteribulin, mirvetuximab soravtansine, ARX788, trastuzumab deruxtecan, trastuzumab duocarmazine, A166, trastuzumab emtansine, DP303c, MRG002, BDC-1001, SHR-A1811, disitamab vedotin, pertuzumab zuvotolimod, patritumab deruxtecan, anetumab ravtansine, BMS-986148, enfortumab vedotin, NBE-002, MRG004, ABBV-3373, sacituzumab govitecan and the like.

The content of the antibody drug in the pharmaceutical composition according to the present invention is 5 to 500 mg/mL, preferably 20 to 200 mg/mL, more preferably 100 to 150 mg/mL, most preferably 120 ± 18 mg/mL, for example, about 110 mg/mL, about 120 mg/mL, or about 130 mg/mL.

The polyclonal antibody contained in the pharmaceutical composition according to the present invention is preferably a serum antibody extracted from serum, such as immune globulin, but is not limited thereto.

For the small molecule compound, any drug that requires a rapid therapeutic or prophylactic effect can be used without limitation. For example, morphine-based analgesics may be used (Thomas et al., 2009). In addition, when the small molecule compound is used for the treatment of tissue necrosis caused by anticancer drugs, it may be used alone or in combination with antidote drugs such as vinca alkaloids and taxane drugs (Kreidieh et al., 2016).

The pharmaceutical composition or formulation according to the present invention contains a surfactant, particularly a poloxamer-based surfactant. Poloxamer is a non-ionic surfactant that has been approved as GRAS (generally recognized as safe substance) by the US FDA and is known to have no adverse effects in acute chest pain even at a dose of 3 g per day (Zarrintaj et al., 2020). Poloxamer-based surfactants are known to have anticancer effects, are also used as an ingredient of hydrogel, and are added at a concentration of 0.1 to 0.5% during cell culture to protect cells from air bubbles. In addition, the poloxamer-based surfactant has a structure represented by Formula (1) or Formula (2), and contains polyethylene oxide (poly-EO) and polypropylene oxide (poly-PO), the names of which are determined based on the content of the ingredient of each of the EO chain and PO chain. The mass contents per molecule of the EO chain is determined within the range of 10% to 80%, and the mass per molecule of the PO chain is determined within the range of 950 to 4,000 Daltons.

### Structure of poloxamer-based surfactants

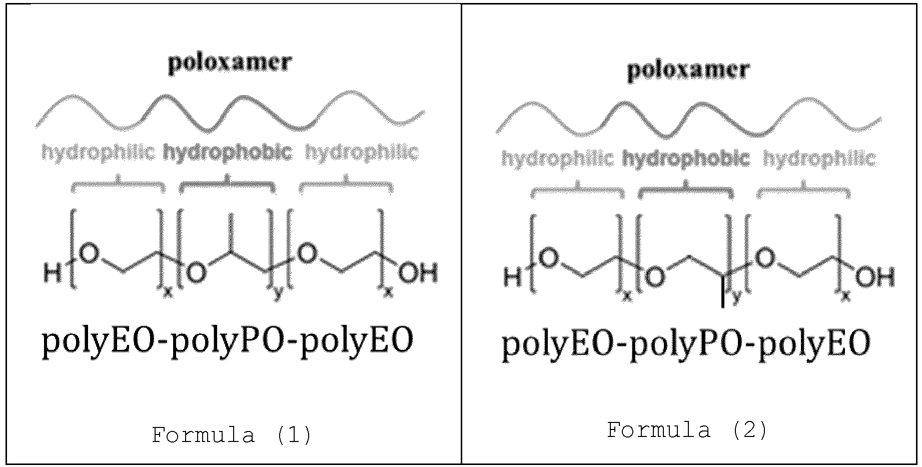

In Formula (1) and Formula (2), x ranges from 2 to 130, and y ranges from 15 to 67, preferably x ranges from 10 to 60, and y ranges from 20 to 40, more preferably x ranges from 10 to 60, and y ranges from 20 to 40, most preferably x is 38 and y is 29.

Preferably, the poloxamer-based surfactant is poloxamer 188, but is not limited thereto. Poloxamer 188, also called "Pluronic F68", refers to a case where the EO chain content is 80% and the molecular weight of the PO chain is 1,750 Daltons. In this case, the molecular weight is 8,750 Daltons. The critical micelle concentration (CMC) of the poloxamer 188 is known to be 0.04 mM (∼0.03%), which is a value much higher than 0.006% and 0.0012% of polysorbate 20 and polysorbate 80, respectively, which are similar nonionic surfactants.

Poloxamer 188 is used in several types of antibody drugs and protein drugs. For example, Poloxamer 188 is used in 0.05% for Enspryng^{™} (Roche), 0.02% for Gazyva^{™} (Roche - Sobi, Sweden), 0.05% for Hemlibra^{™} (Roche), 0.15% for Danyelza^{™} (Y-mAbs), 0.8% for Orencia^{™} (BMS), 0.12% for Nuwiq (Octaphama), and 0.2% for Accretropin (Cangene) (Strichley & Lambert, 2021) .

The concentration of the poloxamer-based surfactant, preferably poloxamer 188, in the pharmaceutical composition according to the present invention is 0.001% to 1.5% (w/v), preferably 0.01% to 1.0% (w/v), more preferably 0.02% to 0.8% (w/v), most preferably 0.05% to 0.5% (w/v), but is not limited thereto.

In one aspect, the present invention is directed to a pharmaceutical composition containing a hyaluronidase and a poloxamer-based surfactant, while containing no drug.

In addition, the pharmaceutical composition according to the present invention may further contain one or more substances selected from the group consisting of buffers and stabilizers.

Any buffer may be used as the buffer contained in the composition according to the present invention without limitation as long as it has a pH of 4 to 8, preferably 5 to 7, and the buffer preferably includes at least one selected from the group consisting of malate, formate, citrate, acetate, propionate, pyridine, piperazine, cacodylate, succinate, 2-(N-morpholino)ethanesulfonic acid (MES), histidine, Tris, bis-Tris, phosphate, ethanolamine, carbonate, piperazine-N,N'-bis(2-ethanesulfonic acid), (PIPES), imidazole, BIS-TRIS propane, BES (N,N-bis(2-hydroxyethyl)-2-aminoethanesulfonic acid), MOPS (3-(N-morpholino)propanesulfonic acid), HEPES (hydroxyethyl piperazine ethane sulfonic acid), pyrophosphate, and triethanolamine, more preferably, histidine buffer, e.g., L-histidine/HCl or acetate buffer, but is not limited thereto.

The concentration of the buffer may be 0.001 to 200 mM, preferably 1 to 50 mM, more preferably 5 to 40 mM, and most preferably 10 to 30 mM.

Any substance commonly used in the art to stabilize proteins may be used without limitation as the stabilizer in the composition according to the present invention. For example, preferably, the stabilizer may include at least one selected from the group consisting of carbohydrates, sugars or hydrates thereof, sugar alcohols and hydrates thereof, and amino acids.

The carbohydrate, sugar or sugar alcohol used as the stabilizer includes at least one selected from the group consisting of trehalose or hydrates thereof, sucrose, saccharin, glycerol, erythritol, threitol, xylitol, arabitol, ribitol, mannitol, sorbitol, galactitol, fucitol, iditol, inositol, volemitol, isomalt, maltitol, polyglycitol, cyclodextrin, hydroxypropyl beta-cyclodextrin, and glucose, and
the amino acid includes at least one selected from the group consisting of glutamine, glutamic acid, glycine, lysine, lysyl-lysine, leucine, methionine, valine, serine, selenomethionine, citrulline, arginine, asparagine, aspartic acid, ornithine, isoleucine, taurine, theanine, threonine, tryptophan, tyrosine, phenylalanine, proline, pyrrolysine, histidine, and alanine, but is not limited thereto, and is preferably methionine.

The concentration of the sugar or sugar alcohol used as a stabilizer in the pharmaceutical composition according to the present invention is 0.001 to 500 mM, preferably 100 to 300 mM, more preferably 150 to 250 mM, most preferably 180 to 230 mM, specifically, about 210 mM.

In addition, the concentration of the amino acid used as the stabilizer in the pharmaceutical composition according to the present invention is 1 to 100 mM, preferably 3 to 30 mM, more preferably 5 to 25 mM, most preferably 7 to 20 mM, specifically, about 8 to 15 mM.

In one embodiment, the pharmaceutical composition according to the present invention may contain 50 to 350 mg/mL of an antibody, such as an anti-HER2 antibody or immune checkpoint antibody, 100 to 20,000 units/mL of a PH20 variant, 0.001% to 1.5% (w/v) of a poloxamer-based surfactant, 0.001 to 200 mM histidine buffer providing a pH of 5.5 ± 2.0, and 10 to 400 mM α,α-trehalose, and optionally contains 1 to 50 mM methionine.

In a more specific embodiment, the pharmaceutical composition according to the present invention contains 120 ± 18 mg/mL of an anti-HER2 antibody or immune checkpoint antibody, 1,500 to 12,000 units/mL of a PH20 variant, 0.1% to 0.5% (w/v) of a poloxamer-based surfactant, 10 to 30 mM histidine buffer providing a pH of 5.5 ± 2.0, and 180 to 230 mM α,α-trehalose, and may optionally further contains 8 to 15 mM methionine.

The pharmaceutical composition according to the present invention may be administered by intravenous injection, subcutaneous injection, intramuscular injection, intraperitoneal injection, endothelial administration, topical administration, intranasal administration, intrapulmonary administration, intrarectal administration, or the like, is preferably subcutaneously administered by subcutaneous injection, and is more preferably an injectable formulation for subcutaneous injection.

Accordingly, in another aspect, the present invention is directed to a formulation containing the pharmaceutical composition according to the present invention, preferably a formulation for subcutaneous administration.

The formulation for subcutaneous administration is preferably an injectable formulation, may be provided in a ready-to-inject form immediately administered without an additional dilution process, and may be packaged in a pre-filled syringe, glass ampoule, plastic container, wearable device or auto-injector.

The wearable device is also called "wearable injector", and examples of the wearable device include, but are not limited to, products from West Pharmaceutical Services, Inc., Enable Injections, Inc., Ypsomed, Weibel CDS,
Subcuject, Eitan Medical, and Sonceboz (Wearable Injectors, 2019) .

In addition, the present invention is directed to a method for treating a disease using the pharmaceutical composition or formulation according to the present invention.

There is no particular limitation as to the disease that can be treated by the pharmaceutical composition or formulation according to the present invention. There is no limitation as to any disease that can be treated with the drug used in combination with PH20 or a PH20 variant according to the present invention.

Examples of the disease that can be treated with the pharmaceutical composition or formulation according to the present invention include, but are not limited to, cancer and autoimmune diseases.

The cancers or carcinomas that can be treated by the pharmaceutical composition or formulation according to the present invention are not particularly limited and include both solid cancers and blood cancers. The cancer may be selected from the group consisting of skin cancer such as melanoma, liver cancer, hepatocellular carcinoma, gastric cancer, breast cancer, lung cancer, ovarian cancer, bronchial cancer, nasopharyngeal cancer, laryngeal cancer, pancreatic cancer, bladder cancer, colorectal cancer, colon cancer, uterine cervical cancer, brain cancer, prostate cancer, bone cancer, thyroid cancer, parathyroid cancer, renal cancer, esophageal cancer, biliary tract cancer, testis cancer, rectal cancer, head and neck cancer, cervical spinal cancer, ureteral cancer, osteosarcoma, neurocytoma, fibrosarcoma, rhabdomyosarcoma, astrocytoma, neuroblastoma and neuroglioma, but is not limited thereto. Preferably, the cancer that can be treated by the pharmaceutical composition or formulation according to the present invention may be selected from the group consisting of stomach cancer, colorectal cancer, breast cancer, lung cancer and renal cancer, but is not limited thereto.

Examples of the autoimmune disease that can be treated with the pharmaceutical composition or formulation according to the present invention include, but are not limited to, rheumatoid arthritis, asthma, psoriasis, multiple sclerosis, allergic rhinitis, Crohn's disease, ulcerative colitis, systemic lupus erythematosus, type I diabetes, inflammatory bowel disease (IBD), and atopic dermatitis.

In addition, the present invention is directed to a method of treating a disease, wherein the pharmaceutical composition or formulation according to the present invention is administered to a subject in need of treatment, and the use of the pharmaceutical composition or formulation according to the present invention for the preparation of a drug for treating a disease.

Unless otherwise defined, technical and scientific terms used herein have meanings commonly understood by those skilled in the art to which the present invention pertains. In addition, repeated description of the same technical configuration and operation as in the prior art will be omitted.

### Example

Hereinafter, the present invention will be described in more detail with reference to examples. However, it will be obvious to those skilled in the art that these examples are provided only for illustration of the present invention and should not be construed as limiting the scope of the present invention.

### Example

### Example 1. Formulation development: test for stability of trastuzumab subcutaneous injection formulation containing poloxamer 188

As shown in Table 6 below, five types of trastuzumab subcutaneous injection formulations were prepared. Formulations 1 to 5 commonly contain 120 mg/mL of trastuzumab, 20 mM histidine/histidine-HCl (pH 5.5), 210 mM trehalose, and 2,000 units/mL of hyaluronidase. Regarding the hyaluronidase used herein, HP46 was used for Formulations 1 to 3 and wild-type recombinant PH20 (rHuPH20) was used for Formulations 4 and 5. In addition, all formulations excluding Formulation 3 contain 10 mM methionine. Regarding the surfactant, 0.04% Polysorbate 20 was included in Formulation 1 and Formulation 4, and 0.2% Poloxamer 188 was in Formulations 2, 3 and 5.

**Table 6. Composition of test formulations**

| | **Formulation 1** | **Formulation 2** | **Formulation 3** | **Formulation 4** | **Formulation 5** |
|---|---|---|---|---|---|
| **Antibody** | Trastuzumab 120 mg/mL | Trastuzumab 120 mg/mL | Trastuzumab 120 mg/mL | Trastuzumab 120 mg/mL | Trastuzumab 120 mg/mL |
| **Buffer** | 20 mM histidine/ histidine-HCl | 20 mM histidine/ histidine-HCl | 20 mM histidine/ histidine-HCl | 20 mM histidine/ histidine-HCl | 20 mM histidine/ histidine-HCl |
| **Stabilizer 1** | 210 mM trehalose | 210 mM trehalose | 210 mM trehalose | 210 mM trehalose | 210 mM trehalose |
| **Stabilizer 2** | 10 mM methionine | 10 mM methionine | - | 10 mM methionine | 10 mM methionine |
| **Surfactant** | Polysorbate 20 0.04% | Poloxamer 188 0.2% | Poloxamer 188 0.2% | Polysorbate 20 0.04% | Poloxamer 188 0.2% |
| **pH** | 5.5 | 5.5 | 5.5 | 5.5 | 5.5 |
| **Hyaluronidas e** | HP46 2,000 units/mL | HP46 2,000 units/mL | HP46 2,000 units/mL | rHuPH20 2,000 units/mL | rHuPH20 2,000 units/mL |

In order to determine the stability of each of these formulations, the formulation was stored under harsh conditions of 40 ± 2°C and 60 ± 5% relative humidity (RH), and was analyzed at intervals of 0, 1, 4, and 7 days as follows.

### A. Measurement of content using spectrophotometer

Changes in protein concentration during the stability period were analyzed using a spectrophotometer. The sample was diluted with distilled water to adjust the concentration thereof to 0.4 mg/mL, and the absorbance of the protein at 280 nm was measured using a spectrophotometer. The relative content based on the content of the first test date is shown in Table 7.

**Table 7. Quantitative results (UV) of Formulations 1 to 5**

| **Content of Trastuzumab** | **Test under harsh conditions (40±2°C, RH 60±5%) (Day)** | | | |
|---|---|---|---|---|
| | **0** | **1** | **4** | **7** |
| Test formulation 1 | 100 | 100 | 98 | 100 |
| Test formulation 2 | 100 | 101 | 98 | 99 |
| Test formulation 3 | 100 | 99 | 97 | 100 |
| Test formulation 4 | 100 | 100 | 98 | 101 |
| Test formulation 5 | 100 | 97 | 100 | 95 |

### B. Investigation of impurity ratio of trastuzumab using size-exclusion chromatography

An HPLC system from Shimadzu Prominence, TSK-gel G3000SWXL (7.8 X 300 mm, 5 µm) and TSK guard column (6.0 x 4.0 mm, 7 µm) were used for size-exclusion chromatography analysis. The mobile phase used herein was 0.2M potassium phosphate (pH 6.2) containing 0.25 M potassium chloride. Analysis was performed for 35 minutes in an isocratic separation mode with a flow rate of 0.5 mL/min. The sample was diluted with an analysis solvent to a final concentration of 10 mg/mL, 20 µL of the dilution was injected into the HPLC column, and the absorbance of the column eluate at 280 nm was recorded. The impurity ratio of trastuzumab was calculated from the HPLC chromatogram and the result is shown in Table 8.

**Table 8. Purity of Formulations 1 to 5 (SEC-HPLC)**

| **Aggregate % SEC-HPLC (Purity of Trastuzumab)** | **Test under harsh conditions (40±2°C, RH 60±5%) (Day)** | | | |
|---|---|---|---|---|
| | **0** | **1** | **4** | **7** |
| Test formulation 1 | 0.31 | 0.36 | 0.51 | 0.56 |
| Test formulation 2 | 0.32 | 0.36 | 0.50 | 0.55 |
| Test formulation 3 | 0.32 | 0.36 | 0.52 | 0.59 |
| Test formulation 4 | 0.30 | 0.37 | 0.51 | 0.57 |
| Test formulation 5 | 0.30 | 0.36 | 0.48 | 0.56 |

### C. IEX chromatography of Formulations 1 to 5

The HPLC system from Shimadzu Prominence, TSKgel CMSTAT (4.6 x 100 mm, 7 µm) as the column and TSKgel guard gel CMSTAT (3.2 mm l.D. × 1.5 cm) were used for IEX chromatography analysis. Mobile phase A used herein was 10 mM sodium phosphate (pH 7.5) and mobile phase B used herein was 10 mM sodium phosphate (pH 7.2) containing 0.1M NaCl. The analysis was performed for 55 minutes at a linear concentration gradient of 0 to 30% mobile phase B at a flow rate of 0.8 mL/min. The sample was diluted with mobile phase A to a final concentration to 1.0 mg/mL, 80 µL of the sample was injected into an HPLC column, and the absorbance of the column eluate at 280 nm was recorded. The monomer ratio of trastuzumab was calculated from the HPLC chromatogram and the result is shown in Table 9.

**Table 9. Distribution of antibody charge variants of Formulations 1 to 5(IEX)**

| **IEX-HPLC (Charge variant of Trastuzumab)** | | **Test under harsh conditions (40±2°C, RH 60±5%) (Day)** | | | |
|---|---|---|---|---|---|
| | | **0** | **1** | **4** | **7** |
| Test formulation 1 | Acidic | 24.6 | 24.3 | 23.4 | 25.5 |
| | Main | 64.7 | 62.8 | 55.9 | 49.2 |
| | Basic | 10.7 | 13.0 | 20.7 | 25.3 |
| Test formulation 2 | Acidic | 24.8 | 24.4 | 23.4 | 25.7 |
| | Main | 64.5 | 62.7 | 56.1 | 48.8 |
| | Basic | 10.8 | 13.0 | 20.5 | 25.5 |
| Test formulation 3 | Acidic | 24.7 | 24.2 | 23.6 | 25.7 |
| | Main | 64.5 | 63.0 | 56.0 | 48.8 |
| | Basic | 10.8 | 12.9 | 20.4 | 25.4 |
| Test formulation 4 | Acidic | 24.8 | 24.4 | 23.9 | 26.0 |
| | Main | 64.5 | 63.2 | 56.5 | 50.0 |
| | Basic | 10.8 | 12.5 | 19.6 | 24.1 |
| Test formulation 5 | Acidic | 24.7 | 24.5 | 23.8 | 26.0 |
| | Main | 64.5 | 63.1 | 56.7 | 49.7 |
| | Basic | 10.8 | 12.4 | 19.6 | 24.3 |

### D. Determination of insoluble particulates for Formulations 1 to 5

In order to measure insoluble particulates, 1 mL of the sample was diluted to 30 mL in a measuring instrument, insoluble particulates of the sample were measured using the method described in the General Test Method of an injection agent, and the result is shown in Table 10. In accordance with the criteria for the insoluble particulates of General Test Methods for injection agent, less than 6,000 insoluble particulates of 10 µm or more (NLT 10 µm) and less than 600 insoluble particulates of 25 µm or more (NLT 25 µm) are required.

**Table 10. Results of insoluble particulate test of Formulations 1 to 5**

| **Sub-visible particle (Insoluble matters)** | | **Test under harsh conditions (40±2°C, RH 60±5%) (Day)** | | |
|---|---|---|---|---|
| **Criteria:** NLT 10 µm 6,000 or less NLT 25 µm 600 or less | | **0** | **4** | **7** |
| Test Formulation 1 | NLT 10 µm | 201 | 57 | 195 |
| | NLT 25 µm | 15 | 4 | 10 |
| Test formulation 2 | NLT 10 µm | 141 | 34 | 94 |
| | NLT 25 µm | 4 | 3 | 0 |
| Test formulation 3 | NLT 10 µm | 126 | 39 | 70 |
| | NLT 25 µm | 3 | 3 | 0 |
| Test formulation 4 | NLT 10 µm | 66 | 60 | 111 |
| | NLT 25 µm | 3 | 0 | 3 |
| Test formulation 5 | NLT 10 µm | 45 | 58 | 84 |
| | NLT 25 µm | 4 | 3 | 3 |

### E. Measurement of hyaluronidase enzyme activity of Formulations 1 to 5

Microturbidimetric assay for measuring enzyme activity is a method of measuring the degree of aggregates formed by reaction of hyaluronic acid remaining in the reaction solution with acidified albumin (BSA), based on absorbance. When hyaluronic acid is hydrolyzed by hyaluronidase, the amount of hyaluronic acid reacting with albumin decreases and the absorbance decreases. As a standard, BTH (Sigma) was diluted to 1, 2, 5, 7.5, 10, 15, 20, 30, 50, or 60 unit/mL and injected into each tube. The sample was diluted to 100X, 300X, 600X, 1200X, and 2400X with enzyme diluent buffer (20 mM Tris HCl, pH 7.0, 77 mM NaCl, 0.01% (w/v) bovine serum albumin) and injected into each tube. 3 mg/mL of a hyaluronic acid solution was diluted 10X to a concentration of 0.3 mg/mL to adjust the volume of each fresh tube to 180 µL. 60 µL of the sample containing hyaluronidase was added to the diluted hyaluronic acid solution, followed by mixing and reacting at 37°C for 45 minutes. When the reaction was finished, 50 µL of the enzyme reacted in a 96-well plate and 250 µL of an acidic albumin solution were added to each well, and shaken for 10 minutes, and absorbance was measured at 600 nm using a spectrophotometer.

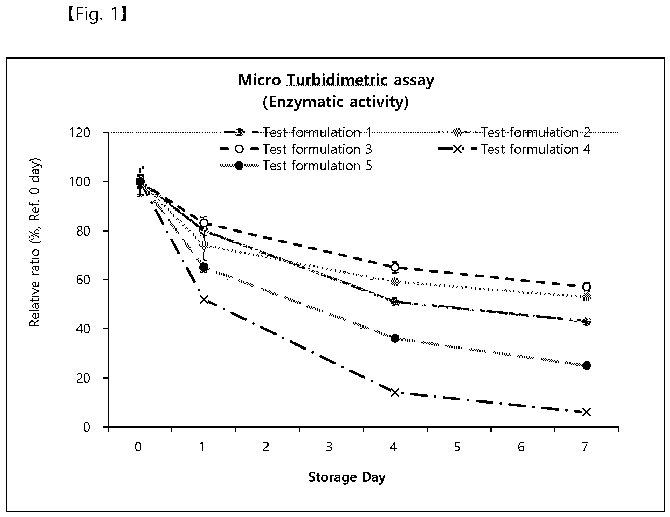

The results of the test of each formulation are shown in FIG. 1.

The results of Example 1 show that the five formulations had no significant changes in sub-visible particles, content, purity, or charge variant and thus have no difference in the antibody stability therebetween. However, compared to the polysorbate 20 0.04% formulation, the poloxamer 188 0.2% formulation had no significant difference in antibody stability, but maintained a much higher hyaluronidase enzyme activity for a longer period of time.

In addition, when the initial hyaluronidase enzyme activity was constant, the HP46-containing formulation maintained higher enzymatic activity for a longer time than the rHuPH20-containing formulation.

### Example 2. Formulation development: harsh stability test of formulation containing hyaluronidase alone

Experiments were conducted to investigate the stability of the formulation containing hyaluronidase alone while containing no other drugs. The formulation containing hyaluronidase alone was prepared as shown in Table 11, and changes in enzyme activity were monitored under harsh conditions of 40 ± 2°C and 60 ± 5% relative humidity, as shown in Table 12.

**Table 11. Composition of test formulations**

| | **Formulation 6** | **Formulation 7** |
|---|---|---|
| **Hyaluronidase** | 3,000 units/mL HP46 | 3,000 units/mL HP46 |
| **Buffer** | 20 mM Sodium phosphate | 20 mM Histidine/Histidine-HCl |
| **Tonicity agent** | 145mM NaCl | 145mM NaCl |
| **Stabilizer** | 10 mM Methionine | 10 mM Methionine |
| **Surfactant** | 0.02% Polysorbate 80 | 0.02% Polysorbate 80 |
| **pH** | **Formulation 6-1:** 6.0, | |
| | **Formulation 6-2:** 6.5, | **Formulation 7-1:** 6.0, |
| | **Formulation 6-3:** 7.0, | **Formulation 7-2:** 6.5, |
| | **Formulation 6-4:** 7.5, | **Formulation 7-3:** 7.0 |
| | **Formulation 6-5:** 8.0 | |

**Table 12. Results of enzymatic activity maintenance test under harsh conditions of Formulations 6 to 7**

| **Test item** | **Relative Hyaluronidase Activity (%) (Activity at 0 time = 100%)** | | | | | |
|---|---|---|---|---|---|---|
| | **0** | **Week 1** | **Week 2** | **Week 4** | **Week 6** | **Week 8** |
| Formulation 6-1 | 100 | 99 | 120 | 86 | 85 | 80 |
| Formulation 6-2 | 100 | 101 | 100 | 82 | 80 | 77 |
| Formulation 6-3 | 100 | 98 | 92 | 68 | 66 | 58 |
| Formulation 6-4 | 100 | 86 | 76 | 46 | 35 | - |
| Formulation 6-5 | 100 | 64 | 43 | 19 | - | - |
| Formulation 7-1 | 100 | 99 | 100 | 87 | 89 | 80 |
| Formulation 7-2 | 100 | 100 | 105 | 93 | 92 | 80 |
| Formulation 7-3 | 100 | 100 | 105 | 89 | 89 | 59 |

As can be seen from Table 12, similarly, Formulations 6 and 7 maintained the enzymatic activity well in the pH range of 6 to 7.

### Example 3. Formulation development: long-term stability test of formulation containing hyaluronidase alone

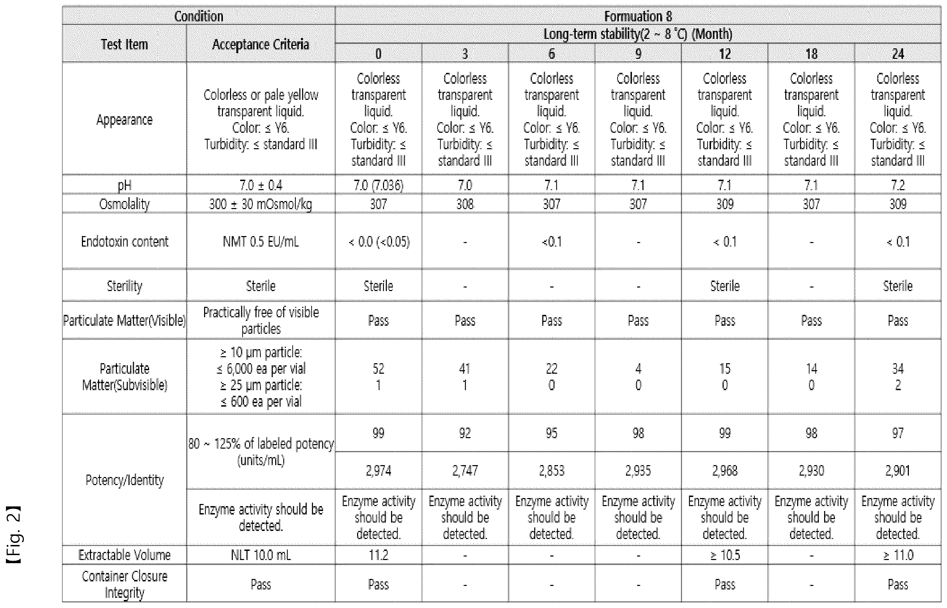

Experiments were conducted to investigate the long-term stability of the formulation containing hyaluronidase alone while containing no other drugs. The formulation 8 containing hyaluronidase alone was prepared with 3,000 units/mL of hyaluronidase (HP46), 10 mM sodium phosphate, 145 mM NaCl, 10 mM methionine, 0.1% human serum albumin, and 0.02% polysorbate 80 (pH 7.0). Changes in appearance, pH, osmolality, endotoxin content, sterility, insoluble matter (particulated matter, visible), insoluble particulate (particulated matter, subvisible), potency/identity, extractable volume, and container-closure integrity thereof were determined at 5 ± 3°C, which is a long-term storage condition, as shown in FIG. 2.

### Example 4. Formulation development: test of stability of trastuzumab subcutaneous injection formulations for each type of surfactant

As shown in Table 13, trastuzumab subcutaneous injection formulations were prepared for each type and concentration of surfactant and the stability of each formulation was determined under harsh conditions. Each formulation contains 120 mg/mL trastuzumab and is composed of 20 mM histidine/histidine-HCl (pH 5.5), 210 mM trehalose, 10 mM methionine, and 2,000 units/mL of hyaluronidase. Hyaluronidase used herein was HP46. Formulations 9-1, 9-2, 9-3, and 9-4 are formulations prepared by adding 0.005%, 0.01%, 0.1%, and 0.2% of polysorbate 20 to the basic ingredients described above, Formulations 10-1, 10-2, 10-3, and 10-4 were formulations prepared by adding polysorbate 80 at 0.005%, 0.01%, 0.1%, and 0.2% thereto, respectively, and Formulations 11-1, 11-2, 11-3, and 11-4 were formulations prepared by adding poloxamer 188 at 0.005%, 0.01%, 0.1% and 0.2% thereto, respectively.

**Table 13. Composition of Test Formulations 9, 10 and 11**

| | **Formulation 9** | **Formulation 10** | **Formulation 11** |
|---|---|---|---|
| **Basic Formulation** | 120 mg/mL Trastuzumab, 20 mM histidine/histidine-HCl, 210 mM trehalose, 10 mM methionine, 2,000 units/mL HP46, pH 5.5 | | |
| **Surfactant** | Polysorbate 20 | Polysorbate 80 | Poloxamer 188 |
| | **Formulation 9-1:** 0.005% | **Formulation 10-1:** 0.005% | **Formulation 11-1:** 0.005% |
| | **Formulation 9-2:** 0.01% | **Formulation 10-2:** 0.01% | **Formulation 11-2:** 0.01% |
| | **Formulation 9-3:** 0.1% | **Formulation 10-3:** 0.1% | **Formulation 11-3:** 0.1% |
| | **Formulation 9-4:** 0.2% | **Formulation 10-4:** 0.2% | **Formulation 11-4:** 0.2% |

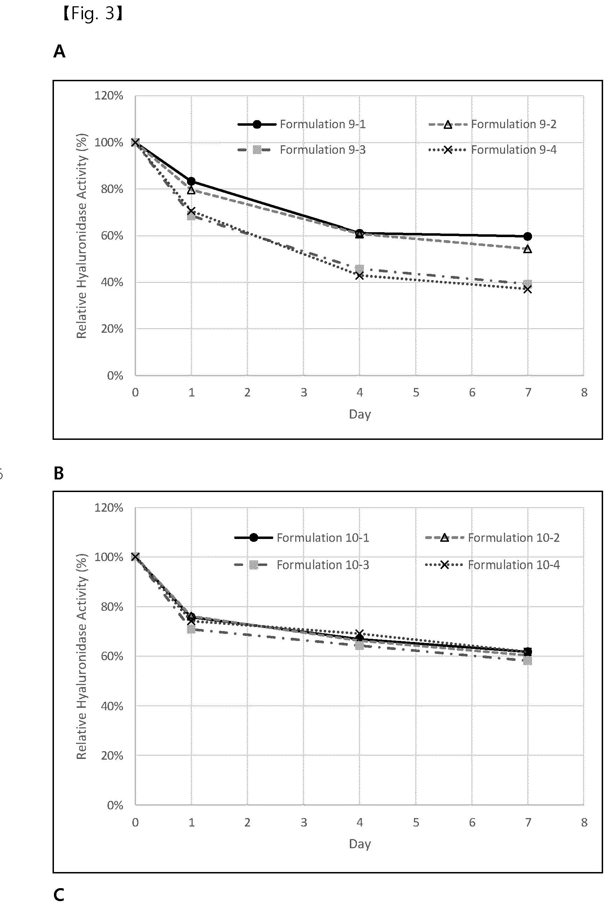

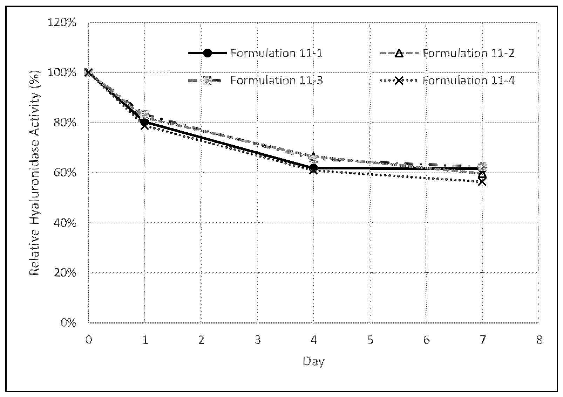

The stability of each trastuzumab subcutaneous injection formulation shown in Table 13 was determined under harsh conditions of 40 ± 2°C and 60 ± 5% relative humidity. Content change was measured in accordance with A. Measurement of content using spectrophotometer in Example 1 and the result is shown in Table 14. The changes in the IEX chromatography were measured using C. IEX chromatography in Example 1 and the result is shown in Table 15. Changes in hyaluronidase enzyme activity were measured in accordance with E. Measurement of hyaluronidase enzyme activity, and the result is shown in A (Formulation 9), B (Formulation 10), and C (Formulation 11) of FIG. 3. The change in activity is shown based on the content or enzyme activity of day 0 as 100% in Table 14 and FIG. 3.

**Table 14. Content changes of Formulations 9, 10, and 11 during harsh stability test period**

| | **Day 0** | **Day 1** | **Day 4** | **Day 7** |
|---|---|---|---|---|
| Formulation 9-1 | 100.0% | 99.3% | 101.2% | 99.7% |
| Formulation 9-2 | 100.0% | 100.1% | 100.3% | 99.9% |
| Formulation 9-3 | 100.0% | 99.3% | 101.0% | 100.9% |
| Formulation 9-4 | 100.0% | 99.0% | 101.3% | 100.9% |
| Formulation 10-1 | 100.0% | 98.9% | 100.1% | 99.0% |
| Formulation 10-2 | 100.0% | 100.2% | 101.1% | 98.3% |
| Formulation 10-3 | 100.0% | 99.1% | 101.4% | 101.0% |
| Formulation 10-4 | 100.0% | 98.3% | 100.8% | 100.0% |
| Formulation 11-1 | 100.0% | 100.3% | 102.3% | 101.3% |
| Formulation 11-2 | 100.0% | 100.6% | 102.1% | 102.3% |
| Formulation 11-3 | 100.0% | 98.2% | 101.0% | 100.0% |
| Formulation 11-4 | 100.0% | 99.0% | 101.5% | 100.2% |

**Table 15. Content changes in IEX chromatography of formulations 9, 10, and 11 during harsh stability test period**

| | **Acidic variants** | | **Main fraction** | | **Basic variants** | |
|---|---|---|---|---|---|---|
| Content (%) | **Day 0** | **Day 7** | **Day 0** | **Day 7** | **Day 0** | **Day 7** |
| Formulation 9-1 | 22.3 | 23.2 | 67.5 | 52.2 | 10.1 | 24.6 |
| Formulation 9-2 | 22.3 | 23.2 | 67.6 | 52.2 | 10.1 | 24.6 |
| Formulation 9-3 | 22.3 | 23.2 | 67.5 | 52.1 | 10.2 | 24.6 |
| Formulation 9-4 | 22.4 | 23.3 | 67.4 | 52.2 | 10.1 | 24.5 |
| Formulation 10-1 | 22.5 | 23.4 | 67.3 | 52.2 | 10.2 | 24.4 |
| Formulation 10-2 | 22.4 | 23.4 | 67.6 | 52.2 | 10 | 24.4 |
| Formulation 10-3 | 22.5 | 23.5 | 67.4 | 52.1 | 10.1 | 24.3 |
| Formulation 10-4 | 22.4 | 23.6 | 67.6 | 52.1 | 10 | 24.3 |
| Formulation 11-1 | 22.4 | 23.9 | 67.5 | 51.8 | 10.1 | 24.4 |
| Formulation 11-2 | 22.3 | 23.8 | 67.6 | 52.0 | 10.1 | 24.2 |
| Formulation 11-3 | 22.4 | 23.8 | 67.5 | 51.9 | 10.1 | 24.3 |
| Formulation 11-4 | 22.4 | 23.9 | 67.5 | 52.0 | 10.1 | 24.2 |

As can be seen from Table 14, there was almost no change in the content of each formulation. As can be seen from Table 15, basic variants increased during the accelerated stability period, but there was no difference between the formulations. This means that there was no difference in the effect on the antibody between the formulations. As can be seen from FIG. 3, the hyaluronidase enzyme activity of Formulation 9 decreased as the content of polysorbate 20, which is a surfactant, increased, and Formulations 10 and 11 had neither increase in the content of surfactant nor decrease in enzyme activity. Formulation 10 containing polysorbate 80 and Formulation 11 containing poloxamer 188 maintained hyaluronidase enzyme activity well despite the increase in surfactant content compared to Formulation 9 containing polysorbate 20.

### Example 5. Formulation development: Test of stability of ocrelizumab subcutaneous injection formulation

Ocrelizumab subcutaneous injection formulations were prepared as shown in Table 16 and the stability of each formulation was determined under harsh conditions. Each formulation contains 30 mg/mL of ocrelizumab and is composed of 20 mM sodium acetate (pH 5.5), 106 mM trehalose, 85 mM sodium chloride, and 2,000 units/mL of hyaluronidase. The hyaluronidases used herein were HP46 and rHuPH20. The surfactants used herein were 0.04% polysorbate 20 and 0.2% poloxamer 188.

**Table 16. Composition of Test Formulation 12**

| | **Formulation 12-1** | **Formulation 12-2** | **Formulation 12-3** | **Formulation 12-4** |
|---|---|---|---|---|
| **Basic Formulation** | 30 mg/mL Ocrelizumab, 20 mM sodium acetate, 106 mM trehalose, 85 mM sodium chloride, pH 5.5 | | | |
| **Hyaluronid ase** | 2,000 units/mL | 2,000 units/mL | 2,000 units/mL | 2,000 units/mL |
| | HP46 | HP46 | rHuPH20 | rHuPH20 |
| **Surfactant** | 0.04% | 0.2% | 0.04% | 0.2% |
| | Polysorbate 20 | Poloxamer 188 | Polysorbate 20 | Poloxamer 188 |

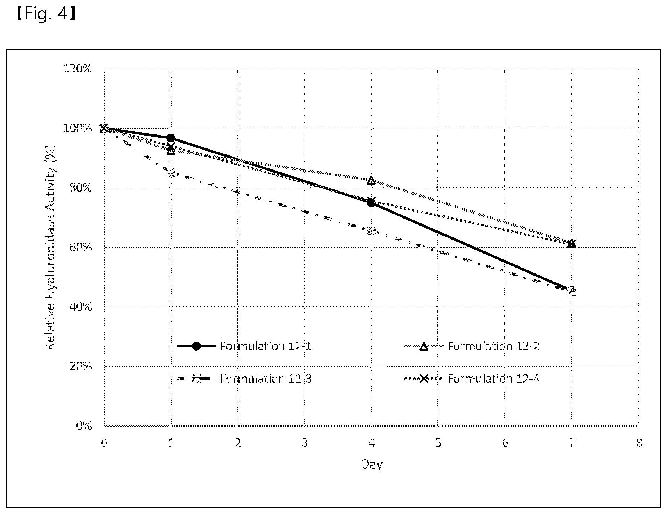

The stability of each ocrelizumab subcutaneous injection formulation shown in Table 16 was determined under harsh conditions of 40 ± 2°C and 60 ± 5% relative humidity. Content change was measured in accordance with A. Measurement of content using spectrophotometer in Example 1 and the result is shown in Table 17. The change in the content of the main fraction in size-exclusion chromatography was measured in accordance with E. size-exclusion chromatography method of Example 1 and the result is shown in Table 18. Changes in hyaluronidase enzyme activity were measured in accordance with the E. Measurement of hyaluronidase enzyme activity, and the result is shown in FIG. 4. The change in activity is shown based on the content or enzyme activity of day 0 as 100% in Tables 17 and 18, and FIG. 4.

**Table 17. Change in content during harsh stability test period of Test Formulation 12**

| | **Day 0** | **Day 1** | **Day 4** | **Day 7** |
|---|---|---|---|---|
| Formulation 12-1 | 100.0% | 99.3% | 100.1% | 100.4% |
| Formulation 12-2 | 100.0% | 99.0% | 99.8% | 97.9% |
| Formulation 12-3 | 100.0% | 100.6% | 101.1% | 99.3% |
| Formulation 12-4 | 100.0% | 99.5% | 99.6% | 98.3% |

**Table 18. Change in size exclusion chromatography during harsh stability test period of Formulation 12**

| | **Day 0** | **Day 1** | **Day 4** | **Day 7** |
|---|---|---|---|---|
| Formulation 12-1 | 100.0% | 100.0% | 99.8% | 99.7% |
| Formulation 12-2 | 100.0% | 100.0% | 99.9% | 99.7% |
| Formulation 12-3 | 100.0% | 100.0% | 99.8% | 99.7% |
| Formulation 12-4 | 100.0% | 100.0% | 99.9% | 99.7% |

As can be seen from Tables 17 and 18, the content of each formulation was maintained without significant change. As can be seen from FIG. 4, the hyaluronidase enzyme activity was maintained at 60% or more when poloxamer 188 was used as a surfactant, and enzyme activity was maintained at 40% or more when polysorbate 20 was used. This means that, although there was no difference in the effect on the antibody between the formulations, the formulation containing poloxamer 188 affected the stability of hyaluronidase enzyme activity. There was no significant difference between HP46 and rHuPH20.

### Example 6. Formulation development: Test of stability of rituximab subcutaneous injection formulation

Rituximab subcutaneous injection formulations were prepared as shown in Table 19 and the stability of each formulation was determined under harsh conditions. Each formulation contains 120 mg/mL of rituximab and was basically composed of 20 mM sodium acetate (pH 5.5), 106 mM trehalose, 85 mM sodium chloride, and 2,000 units/mL of hyaluronidase. The hyaluronidases used herein were HP46 and rHuPH20. The surfactants used herein were 0.04% polysorbate 20 and 0.2% poloxamer 188.

**Table 19. Composition of Test Formulation 13**

| | **Formulation 13-1** | **Formulation 13-2** | **Formulation 13-3** | **Formulation 13-4** |
|---|---|---|---|---|
| **Basic Formulation** | 120mg/mL Rituximab, 20 mM sodium acetate, 106mM trehalose, 85 mM sodium chloride, pH 5.5 | | | |
| **Hyaluronidas e** | 2,000 units/mL | 2,000 units/mL | 2,000 units/mL | 2,000 units/mL |
| | HP46 | HP46 | rHuPH20 | rHuPH20 |
| **Surfactant** | 0.04% | 0.2% | 0.04% | 0.2% |
| | Polysorbate 20 | Poloxamer 188 | Polysorbate 20 | Poloxamer 188 |

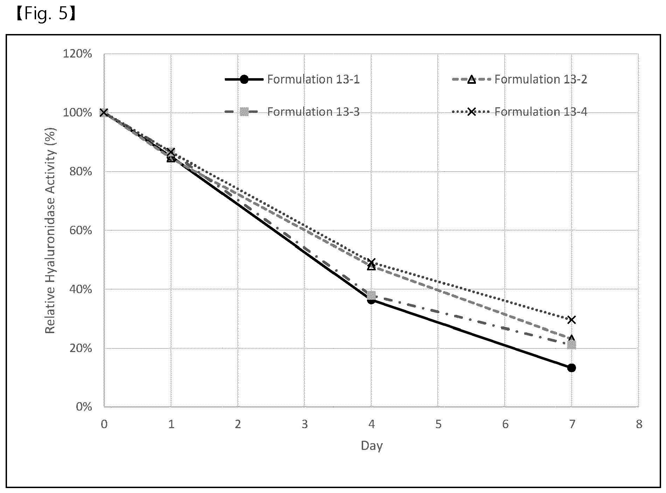

The stability of each rituximab subcutaneous injection formulation shown in Table 19 was determined under harsh conditions of 40 ± 2°C and 60 ± 5% relative humidity. Content change was measured in accordance with A. Measurement of content using spectrophotometer in Example 1 and the result is shown in Table 20. The change in content of the main fraction in size-exclusion chromatography was measured using B. size-exclusion chromatography in Example 1 and the result is shown in Table 21. Changes in hyaluronidase enzyme activity were measured in accordance with E. Measurement of hyaluronidase enzyme activity, and the result is shown in FIG. 5. The change in activity is shown based on the content or enzyme activity of day 0 as 100% in Tables 20 and 21, and FIG. 5.

**Table 20. Content change during harsh stability test period of Formulation 13**

| | **Day 0** | **Day 1** | **Day 4** | **Day 7** |
|---|---|---|---|---|
| Formulation 13-1 | 100.0% | 99.2% | 100.6% | 99.7% |
| Formulation 13-2 | 100.0% | 99.5% | 100.3% | 98.8% |
| Formulation 13-3 | 100.0% | 100.7% | 1020% | 100.9% |
| Formulation 13-4 | 100.0% | 99.7% | 100.9% | 99.4% |

**Table 21. Change in size exclusion chromatography during harsh stability test period of Formulation 13**

| | **Day 0** | **Day 1** | **Day 4** | **Day 7** |
|---|---|---|---|---|
| Formulation 13-1 | 100.0% | 99.9% | 99.6% | 99.3% |
| Formulation 13-2 | 100.0% | 99.9% | 99.6% | 99.3% |
| Formulation 13-3 | 100.0% | 99.9% | 99.6% | 99.3% |
| Formulation 13-4 | 100.0% | 99.8% | 99.6% | 99.3% |

As can be seen from Tables 20 and 21, the content of each formulation was maintained without significant change. As can be seen from FIG. 5, when poloxamer 188 was used as a surfactant, the hyaluronidase enzyme activity was maintained at 20% or more, and in some case, when polysorbate 20 was used as a surfactant, the hyaluronidase enzyme activity was maintained at 20% or less. This means that, although there was no difference in the effect on the antibody between the formulations, the formulation containing poloxamer 188 affected the stability of hyaluronidase enzyme activity. There was no significant difference between HP46 and rHuPH20.

### Example 7. Formulation development: Test of stability of nivolumab subcutaneous injection formulation

Nivolumab subcutaneous injection formulations were prepared as shown in Table 22 and the stability of each formulation was determined under harsh conditions. Each formulation contains 25 mg/mL of nivolumab and was basically composed of 20 mM sodium acetate (pH 5.5), 106 mM trehalose, 85 mM sodium chloride, and 2,000 units/mL of hyaluronidase. The hyaluronidases used herein were HP46 and rHuPH20. The surfactants used herein were 0.04% polysorbate 20 and 0.2% poloxamer 188.

**Table 22. Composition of Test Formulation 14**

| | **Formulation 14-1** | **Formulation 14-2** | **Formulation 14-3** | **Formulation 14-4** |
|---|---|---|---|---|
| **Basic Formulation** | 25 mg/mL Nivolumab, 20 mM sodium acetate, 106 mM trehalose, 85 mM sodium chloride, pH 5.5 | | | |
| **Hyaluronidas e** | 2,000 units/mL | 2,000 units/mL | 2,000 units/mL | 2,000 units/mL |
| | HP46 | HP46 | rHuPH20 | rHuPH20 |
| **Surfactant** | 0.04% | 0.2% | 0.04% | 0.2% |
| | Polysorbate 20 | Poloxamer 188 | Polysorbate 20 | Poloxamer 188 |

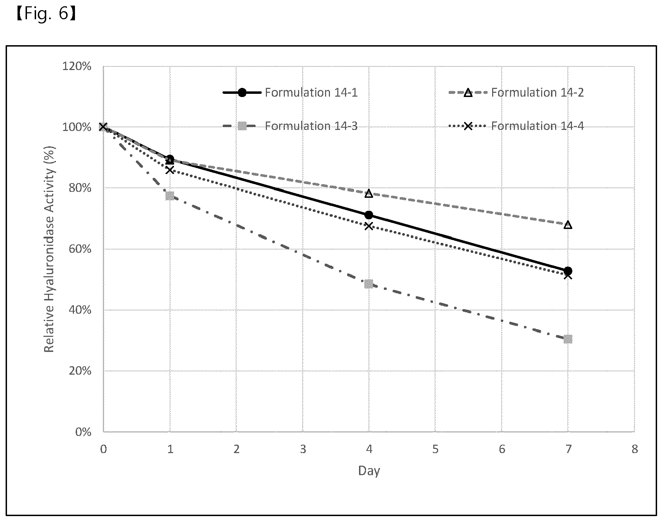

The stability of each nivolumab subcutaneous injection formulation shown in Table 22 was determined under harsh conditions of 40 ± 2°C and 60 ± 5% relative humidity. Content change was measured in accordance with A. Measurement of content using spectrophotometer in Example 1 and the result is shown in Table 23. The change in content of the main fraction in size-exclusion chromatography was measured using B. size-exclusion chromatography in Example 1 and the result is shown in Table 24. Changes in hyaluronidase enzyme activity were measured in accordance with E. Measurement of hyaluronidase enzyme activity, and the result is shown in FIG. 6. The change in activity is shown based on the content or enzyme activity of day 0 as 100% in Tables 22 and 23, and FIG. 6.

**Table 23. Content change during harsh stability test period of Formulation 14**

| | **Day 0** | **Day 1** | **Day 4** | **Day 7** |
|---|---|---|---|---|
| Formulation 14-1 | 100.0% | 100.8% | 99.5% | 100.8% |
| Formulation 14-2 | 100.0% | 100.3% | 99.3% | 100.0% |
| Formulation 14-3 | 100.0% | 99.6% | 99.6% | 99.4% |
| Formulation 14-4 | 100.0% | 99.2% | 99.2% | 99.3% |

**Table 24. change in size exclusion chromatography during harsh stability test period of Formulation 14**

| | **Day 0** | **Day 1** | **Day 4** | **Day 7** |
|---|---|---|---|---|
| Formulation 14-1 | 100.0% | 100.0% | 100.0% | 99.9% |
| Formulation 14-2 | 100.0% | 100.0% | 100.0% | 99.9% |
| Formulation 14-3 | 100.0% | 100.0% | 99.9% | 99.8% |
| Formulation 14-4 | 100.0% | 100.0% | 99.9% | 99.9% |

As can be seen from Tables 23 and 24, the content of each formulation was maintained without significant change. As can be seen from FIG. 6, the enzyme activity of hyaluronidase, HP46, was maintained at 60% or more and the enzyme activity of hyaluronidase, rHuPH20, was maintained at 50% or more, when poloxamer 188 was used as a surfactant, and the enzyme activity of hyaluronidase, HP46, was maintained at 50% or more, and the enzyme activity of hyaluronidase, rHuPH20, was maintained at 30% or less when polysorbate 20 was used. This means that although there was no difference in the effect on the antibody between the formulations, the formulation containing HP46 and poloxamer 188 affected the stability of hyaluronidase enzyme activity.

### Example 8. Formulation development: Test of stability of daratumumab subcutaneous injection formulation

Daratumumab subcutaneous injection formulations were prepared as shown in Table 25 and the stability of each formulation was determined under harsh conditions. Each formulation contains 60 mg/mL of daratumumab and was basically composed of 20 mM sodium acetate (pH 5.5), 106 mM trehalose, 85 mM sodium chloride, and 2,000 units/mL of hyaluronidase. The hyaluronidases used herein were HP46 and rHuPH20. The surfactants used herein were 0.04% polysorbate 20 and 0.2% poloxamer 188.

**Table 25. Composition of Test Formulation 15**

| | **Formulation 15-1** | **Formulation 15-2** | **Formulation 15-3** | **Formulation 15-4** |
|---|---|---|---|---|
| **Basic Formulation** | 60 mg/mL Daratumumab, 20 mM sodium acetate, 106 mM trehalose, 85 mM sodium chloride, pH 5.5 | | | |
| **Hyaluronidas e** | 2,000 units/mL | 2,000 units/mL | 2,000 units/mL | 2,000 units/mL |
| | HP46 | HP46 | rHuPH20 | rHuPH20 |
| **Surfactant** | 0.04% | 0.2% | 0.04% | 0.2% |
| | Polysorbate 20 | Poloxamer 188 | Polysorbate 20 | Poloxamer 188 |

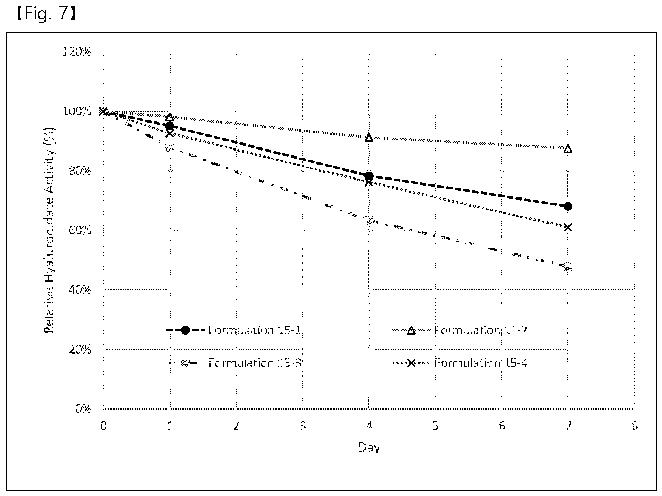

The stability of each daratumumab subcutaneous injection formulation shown in Table 25 was determined under harsh conditions of 40 ± 2°C and 60 ± 5% relative humidity. Content change was measured in accordance with A. Measurement of content using spectrophotometer in Example 1 and the result is shown in Table 26. The change in content of the main fraction in the size-exclusion chromatography was measured using B. size-exclusion chromatography in Example 1 and the result is shown in Table 27. Changes in hyaluronidase enzyme activity were measured in accordance with E. Measurement of hyaluronidase enzyme activity, and the result is shown in FIG. 7. The change in activity is shown based on the content or enzyme activity of day 0 as 100% in Tables 26 and 27, and FIG. 7.

**Table 26. Content change during harsh stability test period of Formulation 15**

| | **Day 0** | **Day 1** | **Day 4** | **Day 7** |
|---|---|---|---|---|
| Formulation 15-1 | 100.0% | 96.8% | 98.9% | 100.3% |
| Formulation 15-2 | 100.0% | 97.8% | 98.7% | 100.1% |
| Formulation 15-3 | 100.0% | 98.1% | 99.3% | 100.4% |
| Formulation 15-4 | 100.0% | 97.6% | 99.6% | 101.2% |

**Table 27. Change in size exclusion chromatography during harsh stability test period of Formulation 15**

| | **Day 0** | **Day 1** | **Day 4** | **Day 7** |
|---|---|---|---|---|
| Formulation 15-1 | 100.0% | 99.8% | 99.3% | 99.1% |
| Formulation 15-2 | 100.0% | 99.6% | 99.1% | 98.9% |
| Formulation 15-3 | 100.0% | 99.8% | 99.3% | 99.1% |
| Formulation 15-4 | 100.0% | 99.9% | 99.4% | 99.2% |

As can be seen from Tables 26 and 27, the content of each formulation was maintained without significant change. As can be seen from FIG. 7, the enzyme activity of hyaluronidase, HP46, was maintained at 80% or more and the enzyme activity of hyaluronidase, rHuPH20, was maintained at 60% or more, when poloxamer 188 was used as a surfactant, and the enzyme activity of hyaluronidase, HP46, was maintained at 60% or more, and the enzyme activity of hyaluronidase, rHuPH20, was maintained at 50% or less when polysorbate 20 was used. This means that, although there was no difference in the effect on the antibody between the formulations, the formulation containing HP46 and poloxamer 188 affected the stability of hyaluronidase enzyme activity.

### Example 9. Formulation development: Test of stability of pembrolizumab subcutaneous injection formulation

Pembrolizumab subcutaneous injection formulations were prepared as shown in Table 28 and the stability of each formulation was determined under harsh conditions. Each formulation contains 25 mg/mL of pembrolizumab and was basically composed of 20 mM sodium acetate (pH 5.5), 106 mM trehalose, 85 mM sodium chloride, and 2,000 units/mL of hyaluronidase. The hyaluronidases used herein were HP46 and rHuPH20. The surfactant used herein was 0.2% poloxamer 188.

**Table 28. Composition of Test Formulation 16**

| | **Formulation 16-1** | **Formulation 16-2** |
|---|---|---|
| **Basic Formulation** | 25 mg/mL Pembrolizumab, 20 mM sodium acetate, 106 mM trehalose, 85 mM sodium chloride, pH 5.5 | |
| **Hyaluronidas e** | 2,000 units/mL | 2,000 units/mL |
| | HP46 | rHuPH20 |
| **Surfactant** | 0.2% | 0.2% |
| | Poloxamer 188 | Poloxamer 188 |

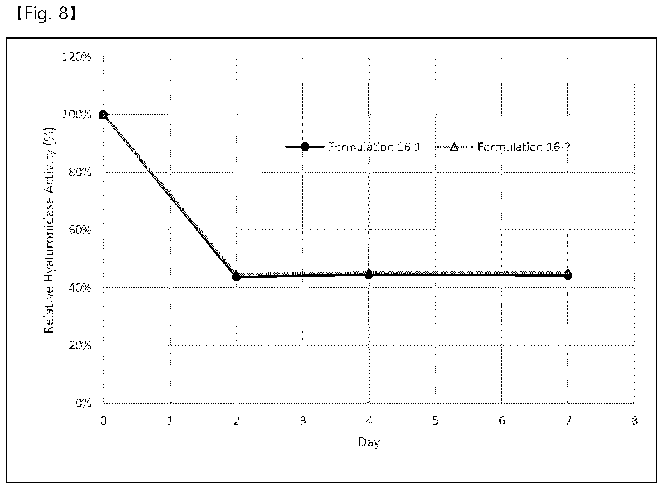

The stability of each pembrolizumab subcutaneous injection formulation shown in Table 28 was determined under harsh conditions of 40 ± 2°C and 60 ± 5% relative humidity. Changes in hyaluronidase enzyme activity were measured in accordance with E. Measurement of hyaluronidase enzyme activity of Example 1, and the result is shown in FIG. 8. The change in activity is shown based on the content or enzyme activity of day 0 as 100% in FIG. 8.

As can be seen from FIG. 8, when poloxamer 188 was used as a surfactant, the enzyme activity was maintained at 40% or more regardless of the type of hyaluronidase.

In summary, the antibody protein drugs prepared from different hyaluronidases and surfactants had no difference in the effects on the antibody between the formulations, but formulations containing HP46 and poloxamer 188 are capable of maintaining stability of hyaluronidase enzyme activity.

### References

Chen, K.J., Sabrina, S., El-Safory, N.S., Lee, G.C., and Lee, C.K. Constitutive expression of recombinant human hyaluronidase PH20 by Pichia pastoris. J Biosci. Bioeng. (2016) 122, 673-678
Hofinger, E.S., Bernhardt, G., and Buschauer, A. Kinetics of Hyal-1 and PH-20 hyaluronidases: comparison of minimal substrates and analysis of the transglycosylation reaction. Glycobiology (2007) 17, 963-971
Kreidieh, F.Y., Moukadem, H.A., and Saghir, N.S.E. Overview, prevention and management of chemotherapy extravasation. World J. Clin. Oncol. (2016) 7, 87-97.
Thomas, J.R., Yocum, R.C., Haller, M.F., and Flament J. The INFUSE-Morphine IIB Study: Use of Recombinant Human Hyaluronidase (rHuPH20) to Enhance the Absorption of Subcutaneous Morphine in Healthy Volunteers. J. Pain Symptom Manag. (2009) 38, 673-682
Strickley, R.G., and Lambert, W.J. A review of formulations of commercially available antibodies. J Pharm. Sci. (2021) 110, 2590-2608
Zarrintaj P., Ramsey J.D., Samadi A., Atoufic Z., Yazdi M.K., Ganjali M.R., Amirabad L.M., Zangene E., Farokhi M., Formela K., Saeb M.R., Mozafari M., and Thomas S. Poloxamer: A versatile tri-block copolymer for biomedical applications. Acta Biomaterialia (2020) 110, 37-67
Wearable Injectors. OnDrugDelivery (2019) 100, 04-91

### INDUSTRIAL APPLICABILITY

The pharmaceutical composition according to the present invention can be used for subcutaneous injection and is capable of maintaining the activity of anticancer drugs, preferably antibody drugs, more preferably antibody drugs such as anti-HER2 antibodies and immune checkpoint antibodies, and PH20 variants for a long period of time due to excellent stability thereof, thus contributing to reducing production costs of subcutaneous formulations and medical costs and being advantageous for patient convenience.

## Claims

1. A pharmaceutical composition comprising:
(a) a drug;
(b) a hyaluronidase; and
(c) a poloxamer-based surfactant,
wherein the hyaluronidase is a wild-type PH20 having a sequence of SEQ ID NO: 1 or a PH20 variant thereof, and
the PH20 variant comprises substitution of one or more amino acid residues selected from the group consisting of T341S, L342W, S343E, I344N, M345T, S347T, M348K, K349E, L352Q, L353A, L354I, D355K, N356E, E359D, I361T, and N363G, and optionally comprises truncation at an N-terminus and/or C-terminus in the wild-type PH20 having a sequence of SEQ ID NO: 1.

2. The pharmaceutical composition according to claim 1, wherein the PH20 variant comprises substitution of one or more amino acid residues selected from the group consisting of L354I and N356E.

3. The pharmaceutical composition according to claim 1, wherein the PH20 variant comprises substitution of one or more amino acid residues at one or more positions selected from the group consisting of an alpha helix and a linker thereof of the PH20 variant having a sequence represented by SEQ ID NO: 1.

4. The pharmaceutical composition according to claim 3, wherein the alpha helix of the wild-type PH20 variant of SEQ ID NO: 1 is an alpha helix 8 (S347 to C381) and the linker is a linker (A333 to R346) between alpha helix 7 and alpha helix 8.

5. The pharmaceutical composition according to claim 4, wherein the corresponding region of the alpha helix and the linker of the wild-type PH20 variant of SEQ ID NO: 1 are T341 to N363, T341 to I361, L342 to I361, S343 to I361, I344 to I361, M345 to I361 or M345 to N363.

6. The pharmaceutical composition according to claim 4, wherein at least one region of the alpha helix 8 (S347 to C381)
and the linker (A333 to R346) between alpha helix 7 and alpha helix 8 of the wild-type PH20 variant of SEQ ID NO: 1 are substituted with one or more amino acid residues in an amino acid sequence of a corresponding region of Hyal1.

7. The pharmaceutical composition according to claim 1, wherein the PH20 variant comprises substitution of L354I and/or N356E, and
further comprises substitution of one or more amino acid residues at one or more positions selected from the group consisting of T341, L342, S343, I344, M345, S347, M348, K349, L352, L353, D355, E359, I361 and N363.

8. The pharmaceutical composition according to claim 7, wherein the PH20 variant comprises substitution of L354I and/or N356E, and
further comprises substitution of one or more amino acid residues at one or more positions selected from the group consisting of T341S, L342W, S343E, I344N, M345T, S347T, M348K, K349E, L352Q, L353A, D355K, E359D, I361T and N363G.

9. The pharmaceutical composition according to claim 7, wherein the PH20 variant comprises substitution of M345T, S347T, M348K, K349E, L352Q, L353A, L354I, D355K, N356E, E359D, and I361T.

10. The pharmaceutical composition according to claim 9, wherein the PH20 variant further comprises substitution of one or more amino acid residues of T341S, L342W, S343E, I344N, and N363G.

11. The pharmaceutical composition according to claim 10, wherein the PH20 variant comprises any one amino acid substitution group selected from the group consisting of the following:
(a) T341S, L342W, S343E, I344N, M345T, S347T, M348K, K349E, L352Q, L353A, L354I, D355K, N356E, E359D and I361T;
(b) L342W, S343E, I344N, M345T, S347T, M348K, K349E, L352Q, L353A, L354I, D355K, N356E, E359D, and I361T;
(c) M345T, S347T, M348K, K349E, L352Q, L353A, L354I, D355K, N356E, E359D, and I361T;
(d) M345T, S347T, M348K, K349E, L352Q, L353A, L354I, D355K, N356E, E359D, I361T, and N363G;
(e) I344N, M345T, S347T, M348K, K349E, L352Q, L353A, L354I, D355K, N356E, E359D, and I361T; and
(f) S343E, I344N, M345T, S347T, M348K, K349E, L352Q, L353A, L354I, D355K, N356E, E359D, and I361T

12. The pharmaceutical composition according to any one of claims 1 to 11, wherein the wild-type PH20 or PH20 variant further comprises deletion of one or more amino acid residues of the C-terminus and the N-terminus.

13. The pharmaceutical composition according to claim 12, wherein the wild-type PH20 or PH20 variant further comprises truncation before an amino acid residue selected from the group consisting of M1 to P42 at the N-terminus to delete one or more amino acid residues.

14. The pharmaceutical composition according to claim 13, wherein the wild-type PH20 or PH20 variant further comprises truncation before an amino acid residue of L36, N37, F38, R39, A40, P41 or P42 at the N-terminus to delete one or more amino acid residues.

15. The pharmaceutical composition according to claim 12, wherein the wild-type PH20 or PH20 variant further comprises truncation after an amino acid residue selected from the group consisting of V455 to L509 at the C-terminus to delete one or more amino acid residues.

16. The pharmaceutical composition according to claim 15, wherein the wild-type PH20 or PH20 variant further comprises truncation after an amino acid residue selected from the group consisting of V455 to S490 at the C-terminus to delete one or more amino acid residues.

17. The pharmaceutical composition according to claim 16, wherein the wild-type PH20 or PH20 variant further comprises truncation after V455, C458, D461, C464, I465, D466, A467, F468, K470, P471, P472, M473, E474, T475, E476, P478, I480, Y482, A484, P486, T488 or S490 at the C-terminus to delete one or more amino acid residues.

18. The pharmaceutical composition according to any one of claims 1 to 17, wherein the wild-type PH20 or PH20 variant further comprises a signal peptide derived from human hyaluronidase-1 (Hyal1), human growth hormone or human serum albumin at the N-terminus.

19. The pharmaceutical composition according to any one of claims 1 to 17, wherein the PH20 variant is further selected from the group consisting of amino acid sequences of SEQ ID NO: 5 to SEQ ID NO: 50.

20. The pharmaceutical composition according to claim 19, wherein the PH20 variant has a sequence represented by SEQ ID NO: 44.

21. The pharmaceutical composition according to claim 1, wherein the drug is a protein drug, antibody, small molecule, aptamer, RNAi, antisense, or cell therapeutic agent.

22. The pharmaceutical composition according to claim 21, wherein the drug is an antibody, a soluble receptor or a fusion protein of a soluble receptor and Fc.

23. The pharmaceutical composition according to claim 22, wherein the antibody binds to at least one antigen selected from the group consisting of 4-1BB, 5T4, integrin, activin, amyloid beta, angiopoietin 1 or 2, angiopoietin analogue 3, B cell maturation antigen (BCMA), B-cell activating factors (BAFF), B7-H3, complement 5, CCR4, CCR5, CCL11, CD2, CD3, CD4, CD6, CD11a, CD16A, CD19, CD20, CD22, CD25, CD27, CD28, CD30, CD32B, CD33, CD38, CD40, CD45, CD46, CD47, CD52, CD56, CD62, CD70, CD73, CD74, CD79b, CD80, CD105, CD123, CD154, CD166, CD262, CD278, CD319, CD326, carcinoembryonic antigen (CEA), CGRP, claudin-18, c-Met, CSF-1, CSF-1 receptors, CTLA4, DLL3, EGF receptors, hemophilia factors, Fc receptors, FGF23, folate receptors, GD2, glucocorticoid-induced TNF receptors (GITR), glypican 3, GM-CSF, HER2, HER3, hepatocyte growth factors (HGF), interferon receptors, interferon gamma, IgE, IGF-1 receptors, interleukin 1, interleukin 2 receptors, interleukin 4, interleukin 4 receptors, interleukin 5, interleukin 5 receptors, interleukin 6, interleukin 6 receptors, interleukin 8, interleukin 12/23, interleukin 13, interleukin 17A, interleukin 17 receptor A, interleukin 23, interleukin 31 receptors, interleukin 36 receptors, lymphocyte-activation gene 3 (LAG3), Lysyl oxidase homolog 2 (LOXL2), mesothelin, mucin-1, mucin-16, nectin 4, nerve growth factors (NGF), OX40, proprotein convertase subtilisin/kexin type 9 (PCSK9), PD-1, PD-L1, phospholipase C, RANKL (receptor activator of nuclear factors kappa B ligand), tyrosine-protein kinase transmembrane receptor (ROR1), sialic acid binding ig-like lectin 15 (Siglec-15), transforming growth factor beta (TGFβ), TIGIT (T-cell immunoreceptor with immunoglobulin and ITIM domain), T-cell immunoglobulin and mucin-domain containing-3 (Tim-3), tissue factors, tissue factor pathway inhibitors (TFPI), TORP-2, tumor necrosis factors (TNF), thymic stromal lymphopoietin (TSLB), large colony stimulating factor 1 receptors (CSF1R), vascular endothelial growth factors (VEGF), VEGF receptors and von Willebrand factors (vWF) .

24. The pharmaceutical composition according to claim 22, wherein the antibody comprises at least one selected from the group consisting of ADG 106, EU101, LVGN6051, urelumab, utomilumab, bebtelovimab, aducanumab, bapinezumab, crenezumab, donanemab, gantenerumab, lecanemab, solanezumab, nesvacumab, evinacumab, enoblituzumab, omburtamab, belimumab, ianalumab, tabalumab, bertilimumab, mogamulizumab, leronlimab, siplizumab, foralumab, muromonab-CD3, otelixizumab, teplizumab, ibalizumab, tregalizumab, zanolimumab, itolizumab, efalizumab, inebilizumab, tafasitamab, tositumomab, ocrelizumab, ofatumumab, rituximab, ublituximab, veltuzumab, epratuzumab, basiliximab, daclizumab, varlilumab, lulizumab pegol, iratumumab, BI-1206, lintuzumab, daratumumab, felzartamab, GEN3014, isatuximab, mezagitamab, CDX-1140, bleselumab, dacetuzumab, iscalimab, lucatumumab, mitazalimab, SEA-CD40, sotigalimab, SAR441344, tegoprubart, dapirolizumab pegol, I-131-apamistamab, AO-176, ligufalimab, magrolimab, alemtuzumab, crizanlizumab, inclacumab, cusatuzumab, oleclumab, milatuzumab, galiximab, carotuximab, adecatumumab, eptinezumab, erenumab, fremanezumab, galcanezumab, TST001, ZL-1211, zolbetuximab, onartuzumab, eculizumab, pozelimab, ravulizumab, lacnotuzumab, PD-0360324, AMB-051, axatilimab, cabiralizumab, emactuzumab, BA3071, ipilimumab, quavonlimab, tremelimumab, zalifrelimab, cetuximab, depatuxizumab, futuximab, imgatuzumab, matuzumab, modotuximab, necitumumab, nimotuzumab, panitumumab, tomuzotuximab, zalutumumab, MK-2060, batoclimab, nipocalimab, rozanolixizumab, burosumab, farletuzumab, dinutuximab, dinutuximab beta, naxitamab, BMS-986156, ragifilimab, gimsilumab, lenzilumab, mavrilimumab, namilumab, otilimab, plonmarlimab, codrituzumab, margetuximab, pertuzumab, trastuzumab, HMBD-001, patritumab, seribantumab, duligotuzumab, ficlatuzumab, rilotumumab, alomfilimab, anifrolumab, emapalumab, FB825, ligelizumab, omalizumab, cixutumumab, dalotuzumab, figitumumab, ganitumab, teprotumumab, bermekimab, canakinumab, gevokizumab, briakinumab, ustekinumab, anrukinzumab, cendakimab, lebrikizumab, tralokinumab, brodalumab, bimekizumab, ixekizumab, secukinumab, brazikumab, guselkumab, mirikizumab, risankizumab, tildrakizumab, nemolizumab, imsidolimab, spesolimab, pascolizumab, CBP201, dupilumab, depemokimab, mepolizumab, reslizumab, benralizumab, clazakizumab, olokizumab, siltuximab, sirukumab, ziltivekimab, levilimab, sarilumab, satralizumab, tocilizumab, HuMax-IL8, abituzumab, favezelimab, fianlimab, GSK2831781, ieramilimab, INCAGN02385, relatlimab, simtuzumab, abagovomab, oregovomab, tanezumab, BMS-986178, GSK3174998, INCAGN01949, ivuxolimab, rocatinlimab, tavolimab, telazorlimab, vonlerolizumab, alirocumab, bococizumab, ebronucimab, evolocumab, frovocimab, ongericimab, tafolecimab, dostarlimab, balstilimab, camrelizumab, cemiplimab, geptanolimab, MEDI0680, nivolumab, pembrolizumab, penpulimab, pidilizumab, prolgolimab, retifanlimab, sasanlimab, serplulimab, sintilimab, spartalizumab, tislelizumab, toripalimab, ezabenlimab, zimberelimab, atezolizumab, avelumab, cosibelimab, sugemalimab, durvalumab, IMC-001, envafolimab, suvratoxumab, denosumab, zilovertamab, NC318, elotuzumab, NIS793, BMS-986207, domvanalimab, EOS-448, etigilimab, ociperlimab, tiragolumab, vibostolimab, surzebiclimab, cobolimab, sabatolimab, TQB2618, concizumab, marstacimab, adalimumab, golimumab, infliximab, certolizumab pegol, conatumumab, tigatuzumab, tezepelumab, gatipotuzumab, cabiralizumab, bevacizumab, brolucizumab, ranibizumab, olinvacimab, icrucumab, ramucirumab, caplacizumab, abrilumab, etrolizumab, vedolizumab, intetumumab, natalizumab, DSP107, RO7122290, cinrebafusp alfa, GEN1042, rozibafusp alfa, GEN1044, obrindatamab, GEN1047, elranatamab, linvoseltamab, teclistamab, epcoritamab, glofitamab, mosunetuzumab, odronextamab, flotetuzumab, vibecotamab, catumaxomab, cibisatamab, TAK-186, talquetamab, ubamatamab, NVG-111, emfizatamab, HPN536, AFM13, blinatumomab, ISB1442, CPO107, AFM24, amivantamab, MCLA-129, SI-B001, emicizumab, Mim8, zenocutuzumab, zanidatamab, tibulizumab, GI-101, vobarilizumab, REGN5668, HX009, cadonilimab, vudalimab, EMB-02, RO7247669, tebotelimab, IBI318, AZD2936, AZD7789, RO7121661, ivonescimab, IBI322, ES101, GEN1046, PRS-344, erfonrilimab, FS118, bintrafusp alfa, HB0036, HLX301, NM21-1480, ozoralizumab, BI836880, faricimab, vanucizumab, navicixizumab, IBI302, naptumomab estafenatox, belantamab mafodotin, DS-7300, MGC018, pivekimab sunirine, praluzatamab ravtansine, coltuximab ravtansine, denintuzumab mafodotin, loncastuximab tesirine, ibritumomab tiuxetan, inotuzumab ozogamicin, epratuzumab-cys-tesirine, moxetumomab pasudotox, brentuximab vedotin, gemtuzumab ozogamicin, vadastuximab talirine, STI-6129, FOR46, lorvotuzumab mertansine, polatuzumab vedotin, tusamitamab ravtansine, telisotuzumab vedotin, rovalpituzumab tesirine, depatuxizumab mafodotin, farletuzumab ecteribulin, mirvetuximab soravtansine, ARX788, trastuzumab deruxtecan, trastuzumab duocarmazine, A166, trastuzumab emtansine, DP303c, MRG002, BDC-1001, SHR-A1811, disitamab vedotin, pertuzumab zuvotolimod, patritumab deruxtecan, anetumab ravtansine, BMS-986148, enfortumab vedotin, NBE-002, MRG004, ABBV-3373, and sacituzumab govitecan.

25. The pharmaceutical composition according to claim 22, wherein the soluble receptor or the soluble receptor contained in the fusion protein of the soluble receptor and Fc is selected from the group consisting of TNF-α soluble receptors, VEGF soluble receptors, CTLA-4, interleukin 1 soluble receptors, and LFA3 soluble receptors.

26. The pharmaceutical composition according to claim 25, wherein the fusion protein of the soluble receptor and Fc is selected from etanercept, aflibercept, conbercept, OPT-302, abatacept, belatacept, rilonacept, inbakicept, alefacept, evorpacept, ontorpacept, TTI-622, sotatercept, dalantercept, acazicolcept, and dazodalibep.

27. A pharmaceutical composition comprising:
a hyaluronidase; and
a poloxamer-based surfactant.

28. The pharmaceutical composition according to claim 1 or 27, wherein the poloxamer-based surfactant is poloxamer 188.

29. The pharmaceutical composition according to claim 1 or 27, wherein the poloxamer-based surfactant is present in an amount of 0.001% to 1.5% (w/v).

30. The pharmaceutical composition according to claim 1 or 27, further comprising at least one selected from the group consisting of a buffer and a stabilizer.

31. The pharmaceutical composition according to claim 30, wherein the buffer comprises at least one selected from the group consisting of malate, formate, citrate, acetate, propionate, pyridine, piperazine, cacodylate, succinate, 2-(N-morpholino)ethanesulfonic acid (MES), histidine, Tris, bis-Tris, phosphate, ethanolamine, carbonate, piperazine-N,N'-bis(2-ethanesulfonic acid), (PIPES), imidazole, BIS-TRIS propane, BES (N,N-bis(2-hydroxyethyl)-2-aminoethanesulfonic acid), MOPS (3-(N-morpholino) propanesulfonic acid), HEPES (hydroxyethyl piperazine ethane sulfonic acid), pyrophosphate, and triethanolamine, and
the stabilizer comprises at least one selected from the group consisting of carbohydrate, sugar or a hydrate thereof, sugar alcohol or a hydrate thereof, and amino acid.

32. The pharmaceutical composition according to claim 31, wherein the carbohydrate, sugar or sugar alcohol comprises at least one selected from the group consisting of trehalose or a hydrate thereof, sucrose, saccharin, glycerol, erythritol, threitol, xylitol, arabitol, ribitol, mannitol, sorbitol, galactitol, fucitol, iditol, inositol, volemitol, isomalt, maltitol, polyglycitol, cyclodextrin, hydroxypropyl beta-cyclodextrin, and glucose, and
the amino acid comprises at least one selected from the group consisting of glutamine, glutamic acid, glycine, lysine, lysyl-lysine, leucine, methionine, valine, serine, selenomethionine, citrulline, arginine, asparagine, aspartic acid, ornithine, isoleucine, taurine, theanine, threonine, tryptophan, tyrosine, phenylalanine, proline, pyrrolysine, histidine, and alanine.

33. The pharmaceutical composition according to claim 30, comprising 50 to 350 mg/mL of an antibody, including an anti-HER2 antibody or an immune checkpoint antibody, 100 to 20,000 units/mL of a PH20 variant, 0.001% to 1.5% (w/v) of a poloxamer-based surfactant, 0.001 to 200 mM histidine buffer providing a pH of 5.5 ± 2.0, and 10 to 400 mM α,α-trehalose, and optionally comprising 1 to 50 mM methionine.

34. The pharmaceutical composition according to claim 33, comprising 120 ± 18 mg/mL of an anti-HER2 antibody or immune checkpoint antibody, 1,500 to 12,000 units/mL of a PH20 variant, 0.1% to 0.5% (w/v) of a poloxamer-based surfactant, 10 to 30 mM histidine buffer providing a pH of 5.5 ± 2.0, and 180 to 230 mM α,α-trehalose, and optionally further comprising 8 to 15 mM methionine.

35. The pharmaceutical composition according to any one of claims 1 to 34, wherein the pharmaceutical composition has a residual enzyme activity of 20% or more at 40 ± 2°C after 7 days.

36. A formulation for subcutaneous administration comprising the pharmaceutical composition according to any one of claims 1 to 35.

37. The formulation according to claim 36, wherein the formulation is provided in a ready-to-inject form immediately administered without an additional dilution process.

38. The formulation according to claim 37, wherein the formulation is packaged in a pre-filled syringe, glass ampoule, plastic container, wearable device or auto-injector.
